# EUROPEAN PATENT APPLICATION

(11) **EP 4 501 270 A1**
(43) Date of publication of application: **05.02.2025**
(21) Application number: 23778066.3
(22) Date of filing: 25.03.2023
(51) Int. Cl.: A61B 34/37

(54) **SURGICAL ROBOT AND CONTROL METHOD**

(30) Priority: 26.03.2022 CN 202210304702; 26.03.2022 CN 202210304701; 15.07.2022 CN 202210833549; 15.07.2022 CN 202210833545
(71) Applicant: Shenzhen Edge Medical Co., Ltd., Shenzhen, Guangdong 518000 (CN)
(72) Inventor: WANG, Shenhui, Shenzhen, Guangdong 518000 (CN); GAO, Yuanqian, Shenzhen, Guangdong 518000 (CN)
(74) Representative: Biallo, Dario
(86) International application number: PCT/CN2023/083915
(87) International publication number: WO 2023/185699

(57) **Abstract**

Disclosed is a surgical robot, comprising input devices (102, 103), robotic arms (210, 220, 230, 240), and a controller (250). Instruments (310, 320, 330, 340) are detachably mounted on the robotic arms (210, 220, 230, 240). The controller (250) interrupts the control of the instruments (310, 320, 330, 340) by the input devices (102, 103) in response to a master/slave control disconnection command. When the robotic arms (210, 220, 230, 240) are actuated to change the orientations of end apparatuses of the instruments (310, 320, 330, 340), the controller (250) controls the motion of the input devices (102, 103) such that the orientations of the input devices (102, 103) conform to the orientations of the end apparatuses of the instruments (310, 320, 330, 340).

## Description

### CROSS-REFERENCE TO RELATED APPLICATION

The present disclosure claims the priority of Chinese Patent Application No. 202210833549.3, filed on July. 15, 2022, and titled "medical apparatus", Chinese Patent Application No. 202210833545.5, filed on July. 15, 2022, 2022, and titled "medical equipment and method for adjusting master-slave posture relation of medical equipment", Chinese Patent Application No. 202210304702.3, filed on March. 26, 2022, and titled "control method for surgical robot, surgical robot and readable storage medium", and Chinese Patent Application No. 202210304701.9, filed on March. 26, 2022, and titled "control method for surgical robot, surgical robot and readable storage medium", the entirety of which are incorporated by reference herein.

### TECHNICAL FIELD

The subject matter herein generally relates to medical instruments, in particular to a master-slave operating surgical robot.

### BACKGROUND

Minimally invasive surgery refers to a surgical method that utilizes modern medical instruments such as laparoscopes, thoracoscopes, and related equipment to perform operations inside the human body cavities. Compared to traditional surgical methods, minimally invasive surgery offers advantages such as smaller incisions, less pain, and faster recovery.

With advancements in technology, minimally invasive medical robotic technology has gradually matured and is being widely applied. Minimally invasive medical robots typically consist of a master console and a slave operating device. Surgeons control the slave operating device by operating the input device of the master console. The slave operating device responds to control commands sent from the master console and executes corresponding surgical operations. The instruments are connected to the driving device of the slave operating device for performing surgical procedures. The distal end of the instrument includes an end effector for executing surgical operations and a joint assembly connected to the end effector that can move with multiple degrees of freedom.

Currently, there is no effective solution for adjusting the orientation of the input device of the master console to realign with the orientation of the end effector of the instrument when they are inconsistent.

### SUMMARY OF THE INVENTION

Aiming at the aforementioned technical problems, a first aspect of the present disclosure provides a surgical robot. The surgical robot comprises an input device; a robotic arm, on which an instrument is detachably mounted; and a controller configured to: suspend control of the instrument by the input device in response to a disconnecting command for master-slave control, and control motion of the input device when the robotic arm is actuated to change an orientation of an end effector of the instrument so that an orientation of the input device follows the orientation of the end effector.

In an embodiment, the robotic arm comprises a parallelogram mechanism and an instrument-holding arm, the instrument is detachably mounted on the instrument-holding arm, and a long shaft of the instrument passes through a remote center of motion defined by the parallelogram mechanism; and the controller is configured to control motion of the input device when the parallelogram mechanism is actuated to rotate the long shaft of the instrument about the remote center of motion so that the orientation of the input device follows the orientation of the end effector of the instrument.

In an embodiment, the robotic arm further comprises an adjustment arm, a distal end of the adjustment arm is connected to a proximal end of the parallelogram mechanism, and the controller is configured to control motion of the input device when the adjustment arm is actuated to change an orientation of the remote center of motion so that the orientation of the input device follows the orientation of the end effector.

In an embodiment, the controller is further configured to resume control of the instrument by the input device in response to an activation command for the master-slave control, and the controller stops controlling the orientation of the input device to be aligned with the orientation of the end effector of the instrument after activation of the master-slave control.

In an embodiment, the input device comprises multiple motors, and the controller is configured to control the multiple motors to rotate in an accelerated manner to move the input device so that the orientation of the input device follows the orientation of the end effector of the instrument.

Aiming at the aforementioned technical problems, a second aspect of the present disclosure provides a surgical robot. The surgical robot comprises an input device and a display device; a first robotic arm and a second robotic arm, an instrument detachably mounted on the first robotic arm, an imaging device detachably mounted on the second robotic arm; and a controller configured to: suspend control of the instrument by the input device in response to a disconnecting command for master-slave control; and control motion of the input device when the second robotic arm is actuated to change an orientation of an end effector of the instrument relative to the imaging device so that an orientation of the input device relative to the display device follows the orientation of the end effector relative to the imaging device.

In an embodiment, the second robotic arm comprises a parallelogram mechanism and an instrument-holding arm, the imaging device is detachably mounted on the instrument-holding arm, and a long shaft of the imaging device passes through a remote center of motion defined by the parallelogram mechanism; and the controller is configured to control motion of the input device when the parallelogram mechanism is actuated to rotate the long shaft of the imaging device about the remote center of motion so that the orientation of the input device relative to the display device follows the orientation of the end effector relative to the imaging device.

In an embodiment, the robotic arm further comprises an adjustment arm, a distal end of the adjustment arm is connected to a proximal end of the parallelogram mechanism, and the controller is configured to control motion of the input device when the adjustment arm is actuated to change an orientation of the remote center of motion so that the orientation of the input device relative to the display device follows the orientation of the end effector relative to the imaging device.

In an embodiment, the controller is further configured to resume control of the instrument by the input device in response to an activation command for the master-slave control, and the controller stops controlling the orientation of the input device to be aligned with the orientation of the end effector of the instrument after activation of the master-slave control.

In an embodiment, the input device comprises multiple motors, and the controller is configured to control the multiple motors to rotate in an accelerated manner to move the input device so that the orientation of the input device f relative to the display device follows the orientation of the end effector relative to the imaging device.

Aiming at the aforementioned technical problems, a third aspect of the present disclosure provides a surgical robot. The surgical robot comprises an input device; a first robotic arm and a second robotic arm, a first instrument detachably mounted on the first robotic arm, a first instrument detachably mounted on the second robotic arm; a controller configured to: switch the input device from controlling the first instrument to controlling the second instrument in response to an instrument-switching command, and control motion of the input device to align an orientation of the input device with an orientation of an end effector of the second instrument.

Aiming at the aforementioned technical problems, a fourth aspect of the present disclosure provides a surgical robot. The surgical robot comprises an input device; a surgical instrument; and a controller configured to: suspend control of the surgical instrument by the input device in response to a disconnecting command for master-slave control; determine whether alignment between the input device and an end effector of the surgical instrument has been performed after initialization of an alignment state between the input device and the surgical instrument; and control an orientation of the input device to follow an orientation of an end effector of the surgical instrument when the alignment has been performed; control the orientation of the input device to be aligned with the orientation of the end effector of the surgical instrument when the alignment has not been performed.

In an embodiment, the controller is further configured to resume control of the surgical instrument by the input device in response to an activation command for the master-slave control, and the controller stops controlling the orientation of the input device to be aligned with the orientation of the end effector of the surgical instrument after activation of the master-slave control.

In an embodiment, the input device comprises multiple motors, and the controller is configured to control the multiple motors to rotate to move the input device.

In an embodiment, the controller is configured to control the multiple motors to rotate in an accelerated manner to move the input device so that the orientation of the input device follows the orientation of the end effector.

In an embodiment, the controller is configured to control the multiple motors to rotate at a uniform speed to move the input device so that the orientation of the input device is aligned with the orientation of the end effector of the surgical instrument.

In an embodiment, the controller is configured to determine whether the orientation of the input device is aligned with the orientation of the end effector of the a surgical instrument; resume control of the a surgical instrument by the input device in response to the activation command for the master-slave control when the orientation of the input device is aligned with the orientation of the end effector of the a surgical instrument; not respond to the activation command for the master-slave control when the orientation of the input device is not aligned with the orientation of the end effector of the a surgical instrument.

Aiming at the aforementioned technical problems, a fifth aspect of the present disclosure provides a surgical robot. The surgical robot comprises an input device, comprising a wrist joint assembly configured to change an orientation of the input device and an elbow joint assembly configured to change a position of the input device; a surgical instrument; and a controller configured to: suspend control of the surgical instrument by the input device in response to a disconnecting command for master-slave control; control motion of the wrist joint assembly in response to motion of the elbow joint assembly or a change in an orientation of the input device so that the orientation of the input device follows an orientation of an end effector of the surgical instrument.

In an embodiment, the controller is further configured to resume control of the surgical instrument by the input device in response to an activation command for the master-slave control, and the controller stops controlling the orientation of the input device to be aligned with the orientation of the end effector of the surgical instrument after activation of the master-slave control.

In an embodiment, the controller is configured to control a motor driving the wrist joint assembly to rotate in an accelerated manner so that the orientation of the input device follows the orientation of the end effector of the surgical instrument.

Aiming at the aforementioned technical problems, a sixth aspect of the present disclosure provides a method for adjusting the master-slave orientation relationship of a surgical robot. The surgical robot comprises an input device, a surgical instrument and a controller. The method comprises: suspending, by the controller, control of the surgical instrument by the input device in response to a disconnecting command for master-slave control, and determining, by the controller, whether alignment between the input device and an end effector of the surgical instrument has been performed; controlling, by the controller, an orientation of the input device to follow an orientation of an end effector of the surgical instrument when the alignment has been performed; controlling, by the controller, the orientation of the input device to be aligned with the orientation of the end effector of the surgical instrument when the alignment has not been performed.

Aiming at the aforementioned technical problems, a seventh aspect of the present disclosure provides a method for adjusting the master-slave orientation relationship of a surgical robot. The surgical robot comprises an input device and a surgical instrument. The input device comprises a wrist joint assembly configured to change an orientation of the input device and an elbow joint assembly configured to change a position of the input device. The method comprises: suspending, by the controller, control of the surgical instrument by the input device in response to a disconnecting command for master-slave control; and controlling, by the controller, motion of the wrist joint assembly in response to motion of the elbow joint assembly or a change in an orientation of the input device so that the orientation of the input device follows an orientation of an end effector of the surgical instrument.

In an embodiment, the method further comprises resuming, by the controller, control of the surgical instrument by the input device in response to an activation command for the master-slave control, and stopping, by the controller, controlling the orientation of the input device to be aligned with the orientation of the end effector of the surgical instrument after activation of the master-slave control.

Aiming at the aforementioned technical problems, an eight aspect of the present disclosure provides control method for a surgical robot. The surgical robot comprises a master console and a slave operating device. The master console comprises a first operating part, the first operating part comprises multiple sets of master joints, and the slave operating device comprises a first instrument with a first end effector. The method comprises determining first slave pose information of the first end effector based on first master pose information of the first operating part according to a mapping between the first operating part and the slave operating device; controlling the first end effector to adjust pose of the first end effector based on the first slave pose information, and acquiring second slave pose information of the first end effector; determining second master pose information of the first operating part based on the second slave pose information according to the mapping between the first operating part and the slave operating device; controlling a first set of master joints among the multiple sets of master joints based on the second master pose information to adjust a pitch and yaw orientations of the first operating part to be aligned with the first end effector; and controlling a second set of master joints among the multiple sets of master joints based on an operator's command to adjust a position of the first end effector to follow a position of the first operating part, and/or to adjust a self-rotation orientation of the first end effector to be aligned with a self-rotation orientation of the first operating part.

In an embodiment, the slave operating device comprises a wrist joint drive device configured to controlling wrist joint motion of an instrument with a wrist joint. The controlling the first end effector to adjust the pose of the first end effector based on the first slave pose information comprises transmitting an operation command to the wrist joint drive device, the operation command indicating the wrist joint drive device to remain stationary; or not transmitting an operation command to the wrist joint drive device, so that the wrist joint drive device remains stationary.

In an embodiment, the controlling the second set of master joints among the multiple sets of master joints based on the operator's command comprises controlling the second set of master joints among the multiple sets of master joints based on the operator's command, wherein change in the second set of master joints does not follow change in the first end effector.

In an embodiment, the method further comprises controlling the change in the first end effector not to follow the change in the second set of master joint.

In an embodiment, the surgical robot further comprises an imaging device and a display device, the determining second master pose information of the first operating part based on the second slave pose information according to the mapping between the first operating part and the slave operating device comprises: determining third slave pose information of the first end effector in the coordinate of the imaging device based on the second slave pose information and the mapping between the base coordinate of the first end effector and the coordinate of the imaging device; determining third master pose information of the end of the first operating part in the coordinate of the display device based on the third slave pose information and the mapping between the coordinate of the imaging device and the coordinate of the display device; determining fourth master pose information of the first operating part in the base coordinate of the master console based on the third master pose information and the mapping between the coordinate of the display device and the base coordinate of the master console; and using the fourth master pose information as the second master pose information.

In an embodiment, the master console further comprises a second operating part, the second operating part comprises multiple sets of master joints, and the slave operating device further comprises a second end effector. The method further comprises: determining fifth slave pose information of the second end effector based on the third slave pose information of the second operating part and the mapping between the second operating part and the slave operating device; controlling the pose adjustment of the second end effector base on the fifth slave pose information and not acquiring sixth slave pose information of the second end effector; or acquiring the sixth slave pose information of the second end effector while not controlling third set of master joints among the multiple sets of master joints of the second operating part based on the sixth slave pose information; and controlling fourth set of master joints of the second operating part based on the operator's command to adjust a position of the second end effector to follow a position of the second operating part and adjust an orientation of the second end effector to be aligned with an orientation of the second operating part.

In an embodiment, the slave operating further comprises a second instrument with a wrist joint. The first instrument does not comprise a wrist joint.

In an embodiment, the controlling the first set of master joints among the multiple sets of master joints based on the second master pose information comprises inversely resolving the second master pose information into a second joint variable; controlling the first set of master joints among the multiple sets of master joints based on the second joint variable.

Aiming at the aforementioned technical problems, a nineth aspect of the present disclosure provides a surgical robot. The surgical robot comprises a master console, a slave operating device and a controller. The master console comprises an operating part. The controller is coupled with the operating part and the slave operating device, and configured to execute the control method for the surgical robot provided by the eighth aspect of the present disclosure.

Aiming at the aforementioned technical problems, a tenth aspect of the present disclosure provides a computer-readable storage medium storing a computer program which is configured to implement the control method for the surgical robot provided by the eighth aspect of the present disclosure when executed by a processer.

Aiming at the aforementioned technical problems, an eleventh aspect of the present disclosure provides control method for a surgical robot. The surgical robot comprises a master console and a slave operating device. The master console comprises a first operating part and a second operating part, a target point is provided between the first operating part and the second operating part, the first operating part comprises a first orientation joint, and the slave operating device comprises a third end effector and an imaging device. The method comprises acquiring a first joint variable of the first orientation joint; controlling the first orientation joint based on the first joint variable to align an orientation of the first operating part with an orientation of the third end effector according to a mapping between the third end effector and the first operating part; controlling the first operating part and the second operating part to change a position and/or a self-rotation orientation of the target point based on an operator's command; and controlling the imaging device to follow the position of the target point and/or controlling the imaging device to be aligned with the self-rotation orientation of the target point based on a mapping between the target point and the imaging device.

In an embodiment, the second operating part comprises a second orientation joint, the slave operating device further comprises a fourth end effector, and the method further comprises: acquiring a third joint variable of the second orientation joint; and controlling the second orientation joint based on the third joint variable to align an orientation of the second operating part with an orientation of the fourth end effector according to a mapping between the fourth end effector and the second operating part.

In an embodiment, the first operating part further comprises a first position joint, the second operating part further comprises a second position joint, and the controlling the first operating part and the second operating part to change the position and/or the self-rotation orientation of the target point based on the operator's command comprises: controlling the first position joint and the second position joint to change the position and/or the self-rotation orientation of the target point based on the operator's command, wherein changes in the first position joint and the second position joint do not follow change in the third end effector.

In an embodiment, the first operating part further comprises a first position joint, the second operating part further comprises a second position joint, and the controlling the first operating part and the second operating part to change the position and/or the self-rotation orientation of the target point based on the operator's command comprises: controlling the first position joint and the second position joint to change the position and/or the self-rotation orientation of the target point based on the operator's command, wherein change in the first position joint does not follow change in the third end effector, and change in the second position joint does not follow change in the fourth end effector.

In an embodiment, the acquiring the first joint variable of the first orientation joint comprises: acquiring fourth slave pose information of the third end effector; determining fifth master pose information of the first operating part based on the fourth slave pose information and the mapping between the first operating part and the slave operating device; determining the first joint variable base on the fifth master pose information.

In an embodiment, the surgical robot further comprises a display device. The determining the fifth master pose information of the first operating part based on the fourth slave pose information and the mapping between the first operating part and the slave operating device comprises: determining fifth slave pose information of the third end effector in the coordinate of the display device based on the fourth slave pose information and the mapping between the base coordinate of the third end effector and the coordinate of the display device; determining sixth master pose information of the first operating part in the coordinate of the display device based on the fifth slave pose information and the mapping between the coordinate of the imaging device and the coordinate of the display device; determining seventh master pose information of the first operating part in the base coordinate of the master console based on the sixth master pose information and the mapping between the coordinate of the display device and the base coordinate of the master console; using the seventh master pose information as the fifth master pose information.

Aiming at the aforementioned technical problems, a twelfth aspect of the present disclosure provides control method for a surgical robot. The surgical robot comprises a master console and a slave operating device. The master console comprises an operating part, the operating part comprises an orientation joint, and the slave operating device comprises an end effector and an imaging device. The method comprises: acquiring a joint variable of the orientation joint; controlling the orientation joint based on the joint variable to align an orientation of the operating part with an orientation of the end effector according to a mapping between the end effector and the operating part; controlling the operating part to change a position and/or a self-rotation orientation of the operating part based on an operator's command; and controlling the imaging device to follow the position of the operating part, and/or controlling the imaging device to be aligned with a self-rotation orientation of the operating part based on a mapping between the operating part and the imaging device.

In an embodiment, the operating part further comprises a position joint. The controlling the operating part to change the position and/or the self-rotation orientation of the operating part based on the operator's command comprises: controlling the operating part to change the position and/or the self-rotation orientation of the operating part based on the operator's command, while change in the position joint does not follow change in the end effector.

Aiming at the aforementioned technical problems, a thirteenth aspect of the present disclosure provides a surgical robot. The surgical robot comprises a master console, a slave operating device and a controller. The master console comprises an operating part. The controller is coupled with the operating part and the slave operating device, and configured to execute the control method for the surgical robot provided by the eleventh or the twelfth aspect of the present disclosure.

Aiming at the aforementioned technical problems, a fourteenth aspect of the present disclosure provides a computer-readable storage medium storing a computer program which is configured to implement the control method for the surgical robot provided by the eleventh or the twelfth aspect of the present disclosure when executed by a processer.

### BRIEF DESCRIPTION OF THE DRAWINGS

FIG. 1 is a top view of a computer-assisted surgical robot for surgery according to an embodiment according to the present disclosure;
FIG. 2 is a schematic view of an instrument according to an embodiment according to the present disclosure;
FIG. 3 is a schematic view of a master console of the surgical robot according to an embodiment according to the present disclosure;
FIG. 4 is a schematic view of a slave operating device of the surgical robot according to an embodiment according to the present disclosure;
FIG. 5 is a schematic view of a slave operating device of the surgical robot according to another embodiment according to the present disclosure;
FIG. 6 is a flow chart of a method for adjusting the master-slave orientation relationship of the surgical robot according to an embodiment according to the present disclosure;
FIG. 7 is a schematic view of an input device of the surgical robot according to an embodiment according to the present disclosure;
FIG. 8 is a schematic view of a robotic arm of the surgical robot according to an embodiment according to the present disclosure;
FIG. 9 is a schematic view of a master wrist of the operating part according to an embodiment according to the present disclosure;
FIG. 10 is a flow chart of a control method for a surgical robot according to an embodiment according to the present disclosure;
FIG. 11 is a flow chart of a control method for a surgical robot according to another embodiment according to the present disclosure.

### DETAILED DESCRIPTION

To facilitate understanding of the present disclosure, a more comprehensive description will be provided below with reference to the accompanying drawings, which illustrate preferred embodiments according to the present disclosure. However, the present disclosure can be implemented in many different forms and is not limited to the embodiments described herein. Rather, these embodiments are provided to make the disclosure of the present disclosure more thorough and comprehensive, and are not intended to limit the present disclosure.

It should be noted that when an element is referred to as being "disposed on" another element, it can be directly on the other element or there may be intervening elements. When an element is considered to be "connected" to another element, it can be directly connected to the other element or there may be intervening elements, and it can also refer to two elements being interconnected through signals. When an element is considered to be "coupled" to another element, it can be directly coupled to the other element or there may be intervening elements, and it can also refer to two elements interacting through signals. The terms "vertical," "horizontal," "left," "right," "upper," "lower," and similar expressions used in this document are for illustrative purposes only and do not represent the only embodiment. It should be understood that these space-related terms are intended to cover different orientations of the device in use or operation, in addition to the orientation depicted in the drawings. For instance, if the device is flipped in the drawings, an element or feature described as being "below" or "under" another element or feature will be oriented "above" that other element or feature. Therefore, the example term "below" can include both upper and lower orientations.

The terms "distal" and "proximal" used in this document serve as directional terms commonly used in the field of interventional medical devices, where "distal" refers to the end away from the surgeon during the surgical procedure, and "proximal" refers to the end close to the surgeon during the surgical procedure.

The term "instrument" is used in the context to describe a surgical robot that is inserted into a patient's body and used to perform surgical or diagnostic procedures. The instrument includes an end effector, which can be a surgical instrument related to performing surgical procedures, such as an electrocautery device, a grasper, a stapler, a cutter, an imaging device (such as an endoscope or ultrasound probe), and similar items. Some instruments used in the embodiments according to the present disclosure further include articulated components (such as joint assemblies) provided for the end effector, allowing the position and orientation of the end effector to be manipulated and moved with one or more mechanical degrees of freedom relative to the instrument axis. Furthermore, the end effector includes functional mechanical degrees of freedom, such as the opening and closing of a grasper. The instrument can further include storage information that can be updated by the surgical system, through which the storage system can provide one-way or two-way communication between the instrument and one or more system components.

Unless otherwise defined, all technical and scientific terms used in this document have the same meanings as commonly understood by those skilled in the technical field of the present disclosure. The terms used in the specification of the present disclosure are for the purpose of describing specific embodiments only and are not intended to limit the present disclosure. The terms "and/or" and "and or" used in this document include any and all combinations of one or more of the listed items.

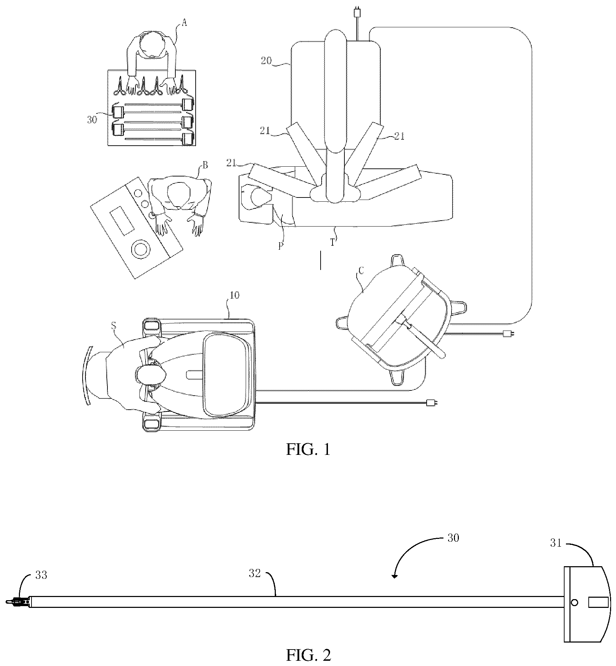

Referring to FIG. 1, an embodiment according to the present disclosure provides a computer-assisted surgical robot. The computer-assisted surgical robot includes a master console 10 and a slave operating device 20. The master console 10 is connected to the slave operating device 20 by remote communication. The slave operating device 50 includes multiple robotic arms 21 and multiple instruments and/or imaging devices are detachably mounted on different robotic arms 21. A surgeon S can remotely operate and control the instruments and/or imaging devices on the master console 10. The master console 10 is configured to send control signals to the slave operating device 20 based on the surgeon S's operations and display images acquired from the slave operating device 20. The surgeon S can observe three-dimensional images inside the patient's body provided by the imaging system through the master console 10. By observing the three-dimensional images inside the patient's body, the surgeon S can control the slave operating device 10 to perform related operations (such as performing surgical procedures or acquiring images inside the patient's body) with an immersive feeling.

The master console 10 is connected to an electronic equipment cart C by remote communication. The electronic equipment cart C is connected to the master console 10 and the slave operating device 20 by remote communication. The electronic equipment cart 30 can include electronic devices such as an energy generation device and an image signal processing device. In the present embodiment, the master console 10, the slave operating device 20, and the electronic equipment cart C are remotely connected via wired Ethernet communication, while remote communication is not limited to wired Ethernet communication and can be other wired methods, including but not limited to serial ports, CAN, RS485, RS232, USB, SPI, etc., or wireless communication methods, including but not limited to WiFi, NB, Zigbee, Bluetooth, RFID, etc..

The computer-assisted surgical robot typically further includes an imaging system that allows the operator to observe the surgical site from outside the patient's body. The imaging system typically includes an imaging device (such as an endoscope) with video and image capture functionality and one or more display devices for displaying the captured images. Generally, the imaging device includes optics that capture images inside the patient's body with one or more imaging sensors (such as CCD or CMOS sensors). The one or more imaging sensors can be placed at the distal end of the imaging device, and the signals generated by the one or more sensors can be transmitted along a cable or wirelessly to be processed and displayed on the display device.

One or more sleeves are connected to the distal end of the robotic arm 21, and the sleeve(s) is inserted into the body of the patient P lying on the surgical table T. The assistant A installs or replaces/reloads the instrument 30 onto the robotic arm 21 according to the surgical conditions. After the instrument 30 is mounted on the robotic arm 21, it is inserted into the patient P's body through the sleeve. The basic surgical team comprises the surgeon S, the assistant A, and the anesthesiologist B.

The instrument 30 can be an electrocautery device, a clamp, a stapler, an ultrasonic scalpel, or other instrument used for performing surgical operations. It can be an imaging device (such as an endoscope) or other surgical instrument for capturing images. Referring to FIG. 2, in some embodiments, the instrument 30 includes a drive unit 31, a long shaft 32, and an end effector 33. The drive unit is used to receive power input from the robotic arm 21 and transmit it to the end effector 33. The end effector 33 can be a device for performing surgery, such as a clamp or an ultrasonic scalpel, or it can be an imaging device, such as an image sensor.

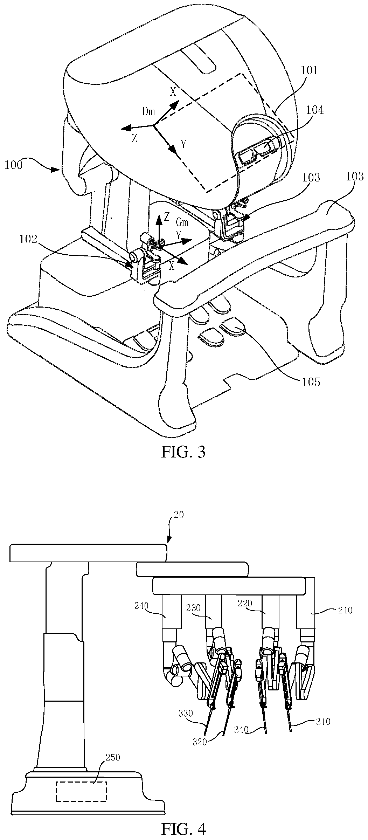

Referring to FIG. 3, a master console is provided by an embodiment according to the present disclosure. The master console 100 includes a display device 101, armrests 103, first and second input devices 102, 103, an observation device 104, and multiple footswitches 105. The two input devices 102, 103 are used to control different instruments or imaging devices. The display device 101 is used to display images captured by the aforementioned imaging system. For instance, the display device 101 is a three-dimensional display device. The surgeon S observes the images displayed on the display device 101 through the observation device 104. The armrests 11 are used for placing the surgeon's arms and/or hands. In some embodiments, the armrests or the observation device 14 can be omitted according to actual needs, which allows direct observation.

The surgeon S controls the motion of the instrument on the slave operating device 20 by operating the first and second input devices 102, 103. The control signal processing system of the master console 100 processes the input signals from the input device 102 and sends control signals to the slave operating device 20. The slave operating device 20 responds to the control signals from the master console 100 and performs corresponding operations, i.e., master-slave control. In some embodiments, the control signal processing system can be provided in the slave operating device 20, for instance, in the base of the slave operating device 20.

Referring to FIG. 4, in some embodiments, the slave operating device 200 includes multiple robotic arms 210, 220, 230, 240, and a controller 250. The multiple robotic arms 210, 220, 230, 240 can have the same or different configurations. Multiple instruments 310, 320, 330, 340 are mounted on the multiple robotic arms 210, 220, 230, 240. Specifically, the first instrument 310 among the multiple instruments is detachably mounted on the first robotic arm 210 the second instrument 320 among the multiple instruments is detachably mounted on the third robotic arm 230, the third instrument 330 among the multiple instruments is detachably mounted on the fourth robotic arm 240, and the imaging device 340 among the multiple instruments is detachably mounted on the second robotic arm 220. In other embodiments, the instruments and imaging devices can be interchangeably mounted on different robotic arms. For instance, the third instrument 330 and the imaging device 320 can be interchangeably mounted. After the interchange, the imaging device 340 is mounted on the third robotic arm 230 and the second instrument 320 is mounted on the second robotic arm 220.

The controller 250 is configured to respond to control signals from the master console 100 or the slave operating device 200, control the joint motions of the robotic arms 210, 220, 230, 240 and the motions of the instruments 310, 320, 330, and the imaging device 340. The controller 250 can be provided in the base of the slave operating device 200. In some embodiments, the controller 250 can be provided in each robotic arm. It is understood that the controller 250 can be provided in the master console 100. In some embodiments, the controller 250 and the aforementioned control signal processing system are the same control device, or they are different control devices respectively provided in the slave operating device 20 and the master console 100.

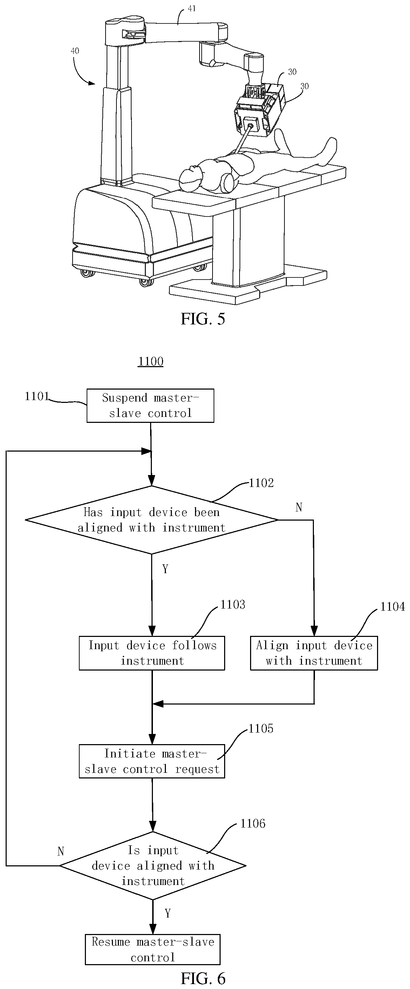

Referring to FIG. 5, in some embodiments, the slave operating device 40 includes one robotic arm 41, and multiple instruments 30 are mounted on the one robotic arm 41. The distal ends of the multiple instruments 40 enter the patient's body through one incision.

In some embodiments, during the master-slave control process, the orientation of the end effectors at the distal ends of the first and second instruments 310, 320 always change to follow the orientation change of the associated first and second input devices 102, 103, so that their orientations are always consistent. The orientation of the first input device 102 refers to the orientation of the coordinate Gm of the proximal handle of the first input device relative to the coordinate Dm of the display device 101. In some situations, the surgeon S sends a command to disconnect the master-slave control to the controller 250 through the first input device 102 and/or the second input device 103. For instance, the surgeon S sends a command to disconnect the master-slave control to the controller 250 by operating a function button of the first input device 102. The controller 250 responds to the command to disconnect the master-slave control and pauses the control of the instrument 310 by the first input device 102, thereby disconnecting the master-slave control. The command to disconnect the master-slave control does not necessarily need to be sent by the surgeon S through the input device 102; it can be sent by the assistant A through the slave operating device 20. Of course, the master-slave control can be disconnected due to other events, such as detecting that the surgeon has left the master console 100.

After the master-slave control is disconnected, changes in the orientation of the first and second input devices 102, 103 and/or changes in the orientation of the end effectors of the instruments and imaging devices cause the orientation of the first and second input devices 102, 103 to no longer be aligned with the orientation of the instruments they control. One method to adjust the orientation relationship between the input devices and the instruments is to activate the master-slave control, i.e., after the master console 100 resumes control of the slave operating device 20, the orientation of the end effectors of the instruments or imaging devices remains unchanged. The controller controls the motion of the first input device 102 to align the orientation of the first input device 102 with the orientation of the end effector of the instrument it controls.

Referring to FIG. 6, in some embodiments, a method for adjusting the orientation relationship between an input device and its associated instrument is provided. Since the adjustment of the orientation relationship between the first input device 102 and its associated instrument is similar to that between the second input device 103 and its associated instrument, the present embodiment and subsequent embodiments, unless specifically stated, use the adjustment of the orientation relationship between the first input device 102 and its associated instrument as an example to illustrate the adjustment of the orientation relationship between an input device and its associated instrument. This is solely for the purpose of clearer and more concise description of the implementation and is not intended to limit the present disclosure. The adjustment of the orientation relationship between the second input device 103 and its associated instrument can refer to the example of adjusting the orientation relationship between the first input device 102 and its associated instrument. In process 1101 of method 1100, the controller 250 suspends the control of the first input device 102 over its associated first instrument 310 or imaging device 320 in response to a master-slave control disconnection command.

In process 1102, after initializing the alignment state between the first input device 102 and the first instrument 310, the controller 250 determines whether alignment has been performed between the first input device 102 and the first instrument 310. If alignment has been performed, the controller 250 proceeds to process 1103, where it controls the motion of the first input device 102 so that the orientation of the first input device 102 follows the orientation of the end effector of the first instrument 310. This orientation -following motion is real-time or synchronous, ensuring that the orientation of the first input device 102 remains consistent with the orientation of the end effector of the instrument 310 in real-time.

In some embodiments, the alignment state between the first input device 102 and the first instrument 310 comprises marking of the alignment state between the first input device 102 and the first instrument 310 by the controller 250. For instance, the controller 250 marks the aligned state as true and the unaligned state as false in software. The initialization of the alignment state between the first input device 102 and the first instrument 310 includes the controller 250 clearing or resetting the alignment state to a default or initial value. In some embodiments, before the controller 250 controls the motion of the first input device 102, when the orientation of the first input device 310 and/or the first instrument 310 change, the controller 250 determines whether the alignment between the first input device 102 and the first instrument 310 has been performed by determining whether the first input device 102 and the first instrument 310 are aligned. If the first input device 102 and the first instrument 310 are aligned when the orientation of the first input device 310 and/or the first instrument 310 change, it is determined that the alignment between the first input device 102 and the first instrument 310 has been performed; otherwise, it is determined that alignment has not been performed. If the alignment between the first input device 102 and the end effector of the first instrument 310 has not been performed, the controller 250 proceeds to process 1104, where it controls the motion of the first input device 102 to align its orientation with the orientation of the end effector of the first instrument 310. This alignment motion is not real-time or synchronous. For instance, the first input device 102 begins the alignment action when the end effector of the first instrument 310 is not moving. Although the orientations of the first input device 102 and the first instrument 310 are not aligned in real-time, their orientations are ultimately aligned. For instance, after determining that the first instrument 310 is first mounted on the robotic arm 210, the controller 250 executes process 1104. Similarly, the same method is applied to adjust the orientation relationship between the second input device 103 and its associated second instrument 320, which ensures that before master-slave control is activated, the orientation of the first input device 102 is already aligned with the orientation of the end effector of the first instrument 310, and the orientation of the second input device 103 is already aligned with the orientation of the second instrument 320. Therefore, the alignment between the orientations of the first and second input devices 102 and the alignment between the orientations of the end effectors of the first and second instruments 310, 320 are not required after master-slave control is activated, improving the efficiency of surgeon operating the computer-assisted surgical robot. Furthermore, in process 1103, since the orientation of the first input device 102 follows the orientation of the end effector of the first instrument 310 in real-time during the master-slave control disconnection, their orientations remain aligned in real-time. Therefore, surgeons can intervene at any point during master-slave control disconnection to activate the master-slave control, further improving the efficiency of surgeon operating the computer-assisted surgical robot.

Referring to FIG. 6, in some embodiments, in process 1105 of activating the master-slave control, the control signal processing system of the master console 100 initiates a master-slave control request to the controller 250. In process 1106, the controller 250 determines whether the orientation of the first input device 102 is aligned with the orientation of the end effector of the first instrument 310. If the orientation of the first input device 102 is not aligned with the orientation of the end effector of the first instrument 310, the controller does not respond to the master-slave control request, and activation of master-slave control fails. If the orientation of the first input device 102 is aligned with the orientation of the end effector of the first instrument 310, the controller responds to the master-slave control request and successfully activates master-slave control, and the first input device 102 resumes control of the first instrument 310, further ensuring the safety of master-slave operation.

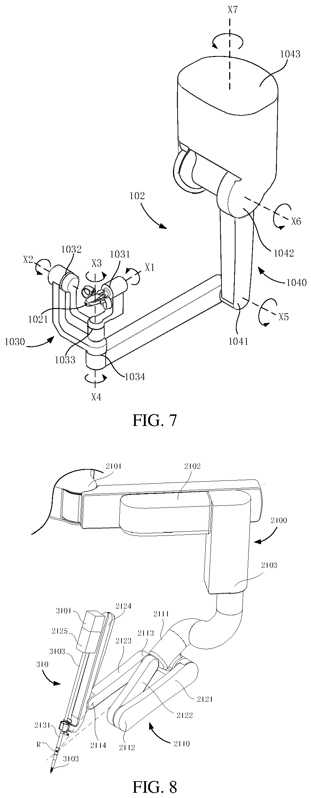

Referring to FIG. 7, in some embodiments, the first input device 102 includes a handle 1021, a wrist joint assembly 1030, and an elbow joint assembly 1040. The handle 1021 is configured for surgeons to hold. The motion of the wrist joint assembly 1030 is configured to change the orientation of the first input device 102, such as the orientation of the handle 1021. The elbow joint assembly 1040 is configured to change the position of the first input device 102, such as the position of the handle 1021.

The wrist joint assembly 1030 includes multiple wrist joints 1031, 1032, 1033, 1034, which are interconnected through L-shaped links for mutual rotational motion. The wrist joint 1031 rotates about its axis line X1, the wrist joint 1032 rotates about its axis line X2, the wrist joint 1033 rotates about its axis line X3, and the wrist joint 1034 rotates about its axis line X4. The rotation of each wrist joint about its axis changes the orientation of the input device 102. In some embodiments, the orientation of the input device 102 includes the orientation of the intersection of axis lines X1, X2, X3, and X4.

The elbow joint assembly 1040 includes multiple elbow joints 1041, 1042, 1043. The elbow joint 1041 rotates about its axis line X5, the elbow joint 1042 rotates about its axis line X6, and the elbow joint 1043 rotates about its axis line X7. The rotation of each elbow joint about its axis changes the position of the input device 102.

The wrist joint assembly 1030 and the elbow joint assembly 1040 comprise multiple motors of the motion of the wrist joint assembly 1030 and the elbow joint assembly 1040. In some embodiments, the controller 250 controls the motion of the wrist joint assembly 1030 by controlling the motion of one or more motors of the wrist joint assembly 1030, thereby enabling the orientation of the first input device 102 and/or the second input device 103 to follow or be aligned with the orientation of the instrument's end effector.

In some embodiments, when the first input device 102 has been aligned with the first instrument 310, the controller 250 controls the motors of the wrist joint 1030 of the first input device 102 to move in an accelerated manner, so that the orientation of the first input device 102 quickly follows the orientation of the end effector of first instrument 310. When the first input device 102 has not been aligned with the first instrument 310, the controller 250 controls the motors of the wrist joint assembly 1030 to move at a uniform speed, allowing the wrist joint assembly 1030 to move uniformly, meaning the handle 1021 moves at a uniform speed, thus avoiding sudden change of the handle 1021's orientation during the alignment process.

In some embodiments, the first input device 102 follows the orientation of its associated first instrument 310 in an accelerated manner, while the second input device 103 is aligned with the orientation of its associated second instrument 320 at a uniform speed.

In some embodiments, during suspension of the master-slave control, when the elbow joint assembly 1040 of the first input device 102 moves, it may cause change in the orientation of the first input device 102. For instance, when the first input device 102 reaches its operational limit, the surgeon S actively moves the elbow joint assembly 1040 by operating the handle 1021 to reposition the first input device 102, or the elbow joint assembly 1040 is moved due to external contact. The orientation of the first input device 102 refers to the orientation of its handle 1021 relative to the display device 101. In response to the motion of the elbow joint assembly 1040 of the first input device 102, the controller 250 controls the motion of the wrist joint assembly 1030, so that the orientation of the input device 102 follows the orientation of the end effector of its associated first instrument 310 in real-time. Thus, during the motion of the elbow joint assembly 1040, the orientation of the first input device 102 remains aligned with the orientation of the end effector of the first instrument 310 in real-time. In some embodiments, the controller 250 can respond to the motion of the elbow joint assembly 1040 by detecting a signal from the encoder of the motor of the elbow joint assembly 1040.

In some embodiments, in response to the motion of the elbow joint assembly 1040, the controller 250 controls the motors of the wrist joint assembly 1030 to move in an accelerated manner, resulting in the wrist joint assembly 1030 moving in an accelerated manner, so that the orientation of the first input device 102 follows the orientation of the instrument's end effector.

In some embodiments, in response to change in the orientation of the first input device 102, the controller 250 controls the motion of the wrist joint assembly 1030, so that the orientation of the first input device 102 follows the orientation of the end effector of first instrument 310.

Referring to FIG. 8, in some embodiments, the robotic arm 210 of the slave operating device comprises an adjustment arm 2100, a parallelogram mechanism 2110, and an instrument-holding arm 2124. The adjustment arm 2100 includes a rotary joint 2101, a horizontal linear joint 2102, and a vertical linear joint 2103, which are connected by connecting rods. The proximal end of the parallelogram mechanism 2110 is connected to the distal end of the adjustment arm 2100. The parallelogram mechanism 2110 includes multiple rotary joints 2111, 2112, 2113, 2114. The rotary joint 2111 is connected to the rotary joint 2112 via a parallelogram mechanism base 2121. The rotary joint 2112 is connected to the rotary joint 2113 via a first connecting rod 2122. The rotary joint 2113 is connected to the rotary joint 2114 via a second connecting rod 2123. The second connecting rod 2123 is connected to the instrument-holding arm 2124 via the rotary joint 2114. A sleeve 2131 is attached to the distal end of the instrument-holding arm 2124. The instrument box 3101 (e.g., a transmission box) of the first instrument 310 is detachably mounted on a driver 2125 on the instrument-holding arm 2124 and receives power input from the driver 2125. The distal end of the long shaft of the first instrument 310 passes through the sleeve 2131, and the end effector 3103 is located at the end of the long shaft.

The first connecting rod 2122, the second connecting rod 2123, the rotary joint 2112, and the rotary joint 2113 of the parallelogram mechanism 2110 mechanically define a parallelogram. The remote center of motion R is located at one vertex of the parallelogram. The rotation axis of the rotary joint 2111 passes through the remote center of motion R, and the long shaft 3103 passes through the remote center of motion R. Therefore, regardless of how the various rotary joints of the parallelogram mechanism 2110 rotate, the long shaft 3102 of the first instrument 310 always moves around the remote center of motion R.

In some embodiments, after suspension of master-slave control, the motion of the parallelogram mechanism 2110, such as the rotation of one or more of the rotary joints 2111, 2112, 2113, 2114 of the parallelogram mechanism, causes the long shaft 3103 of the instrument 310 to move around the remote center of motion R, and the orientation of the end effector 3103 change. For instance, when an assistant A directly moves the parallelogram mechanism 2110, causing a change in the orientation of the end effector 3103. In response to the motion of the parallelogram mechanism 2110, the controller 250 controls the motion of the wrist joint assembly 1030 of the first input device 102 associated with the first instrument 310, so that the orientation of the first input device 102 follows the orientation of the end effector 3103 of the first instrument 310 in real-time.

In some embodiments, in response to the motion of the parallelogram mechanism 2110, the controller 250 controls the motors of the wrist joint assembly 1030 to move in an accelerated manner, so that the orientation of the first input device 102 quickly follows the orientation of the end effector 3103 of the first instrument 310.

In some embodiments, the imaging device 340 is detachably mounted on a driver of the second robotic arm 220, and the first instrument 310 is detachably mounted on the first robotic arm 210. The first robotic arm 210 and the second robotic arm 220 have the same configuration. After the master-slave control is suspended, the motion of the parallelogram mechanism 2110 of the second robotic arm 220 causes a change in the orientation of the end effector (such as an image sensor) at the distal end of the imaging device 340, thereby causing a change in the orientation of the end effector 3103 of the first instrument 310 relative to the end effector of the imaging device 340. At this time, in response to the motion of the parallelogram mechanism 2110 of the second robotic arm 220, the controller 250 controls the motion of the wrist joint assembly 1030 of the first input device 102 associated with the first instrument 310, so that the orientation of the first input device 102 relative to the display device 101 follows the orientation of the end effector 3103 of the first instrument 310 relative to the imaging device 330. In some embodiments, in response to a change in the orientation of the end effector 3103 of the first instrument 310 relative to the end effector of the imaging device 340, the controller 250 controls the motion of the wrist joint assembly 1030 of the first input device 102 associated with the first instrument 310, so that the orientation of the first input device 102 relative to the display device 101 follows the orientation of the end effector 3103 of the first instrument 310 relative to the imaging device 330.

In some embodiments, the motion of the adjustment arm 2100 causes the entire parallelogram mechanism 2110 and the instrument-holding arm 2124 to move, thereby changing the position and orientation of the remote center of motion R and the end effector 3103 of the instrument 310. For instance, at least one of the rotation joint 2101, the horizontal linear joint 2102, and the vertical linear joint 2103 of the adjustment arm 2100 are moved, or the assistant A drags the adjustment arm 2100 to adjust the position of the remote center of motion R. After the master-slave control is suspended, in response to the motion of the adjustment arm 2100, the controller 250 controls the motion of the wrist joint assembly 1030 of the input device 102, so that the orientation of the first input device 102 follows the orientation of the end effector 3103 of the first instrument 310 in real-time.

In some embodiments, the imaging device 340 is detachably mounted on the driver of the second robotic arm 220, and the first instrument 310 is detachably mounted on the first robotic arm 210. The second robotic arm 220 and the first robotic arm 210 have the same configuration. After the master-slave control is suspended, the motion of the adjustment arm 2100 of the second robotic arm 220 causes a change in the orientation of the end effector at the distal end of the imaging device 340, thereby causing a change in the orientation of the end effector 3103 of the first instrument 310 relative to the end effector (such as an image sensor) of the imaging device 330. At this time, in response to the motion of the adjustment arm 2100 of the second robotic arm 220, the controller 250 controls the motion of the wrist joint assembly 1030 of the first input device 102 associated with the first instrument 310, so that the orientation of the first input device 102 relative to the display device 101 follows the orientation of the end effector 3103 of the first instrument 310 relative to the imaging device 330.

In some embodiments, after the surgeon S operates the pedal 105 to issue an instrument-switching command, the controller 250 switches the instrument associated with the first input device 102 in response to the instrument-switching command. For instance, if the first input device 102 is currently associated with the first instrument 310, i.e. the first input device 102 controls the motion of the first instrument 310, after the controller 250 responds to the instrument-switching command, the controller 250 switches the association of the first input device 102 to the second instrument 330, and the first input device 102 controls the motion of the second instrument 330 after the switching. In response to the instrument-switching command that switches the association of the first input device 102 to the second instrument 330, the controller 250 moves the wrist joint assembly 1030 of the first input device 102 to align the orientation of the first input device 102 with the orientation of the second instrument 330 which is newly associated with the first input device 102. After the first input device 102 is switched to be associated with the second instrument 330 and the master-slave control is activated, the first input device 102 controls the motion of the second instrument 330. Moreover, since the orientation of the first input device 102 has been aligned with the orientation of the second instrument 330 before the activation of the master-slave control, the alignment between the orientation of the input device 102 with the orientation of the second instrument 330 is not required after the activation, which improves the efficiency of surgical robot operation. In some embodiments, the instrument-switching command is issued by the surgeon S operating a side pedal.

In some embodiments, in response to the instrument-switching command, the controller 250 moves the motor of the wrist joint assembly 1030 of the first input device 102 at a uniform speed, so that the orientation of the first input device 102 is aligned with the orientation of the instrument which is newly associated with the first input device 102.

In some embodiments, the first instrument 310 is detachably mounted on the first robotic arm 210, and the imaging device 340 is detachably mounted on the second robotic arm 220. In some situations, such as when adjusting the surgical field of view, it is necessary to replace the imaging device 340 from the second robotic arm 220 to the third robotic arm 230. This can be done by swapping the imaging device 340 mounted on the second robotic arm 220 with the second instrument 330 mounted on the third robotic arm 230, or by replacing the imaging device 340 mounted on the second robotic arm 220 to the third robotic arm 230, leaving the second robotic arm 220 unloaded without any instrument attached.

After the imaging device 340 is replaced from the second robotic arm 220 to the third robotic arm 230, the position and orientation of the end effector 3103 of the first instrument 310 on the first robotic arm 210 are switched from referencing the coordinate of the imaging device 340 on the second robotic arm 220 to referencing the coordinate of the imaging device 330 on the third robotic arm 230, changing the pose relationship of the end effector 3103 of the instrument 310 on the first robotic arm 210 relative to the reference coordinate.

At this time, in response to the replacement of the imaging device 340 from the second robotic arm 220 to the third robotic arm 230, the controller 250 controls the motion of the wrist joint assembly 1030 of the first input device 102, so that the orientation of the first input device 102 relative to the display device 101 is aligned with the orientation of the end effector 3103 of the first instrument 310 relative to the imaging device 340 mounted on the third robotic arm 230. In some embodiments, in response to the replacement of the imaging device 340 from the second robotic arm 220 to the third robotic arm 230, the controller 250 controls the wrist joint assembly 1030 of the first input device 102 to move at a uniform speed, so that the orientation of the first input device 102 relative to the display device 101 is aligned with the orientation of the end effector 3103 of the first instrument 310 relative to the imaging device 340 mounted on the third robotic arm 230. In some embodiments, the controller 250 controls the motion of the wrist joint assembly 1030 only after the orientation of the imaging device 340 is locked, so that the orientation of the first input device 102 relative to the display device 101 is aligned with the orientation of the end effector 3103 of the first instrument 310 relative to the imaging device 340 mounted on the third robotic arm 230.

After the orientation of the first input device 102 is aligned with the orientation of the first instrument 310, the surgeon activates the master-slave control and observes the surgical environment through images acquired from the imaging device 340 on the third robotic arm 230. Once the master-slave control is activated, realignment between the orientation of the first input device 102 and the orientation of the first instrument 310 is not required, which improves surgical efficiency.

In some embodiments, the imaging device 340 may need to be replaced, for instance, due to issues of the imaging device 340 during the surgery. After replacing the imaging device 340, the orientation of the replaced imaging device 330 is different from the orientation of the imaging device 330 before the replacement, causing the orientation of the end effector of the instrument on other robotic arms relative to the orientation of the replaced imaging device 330 to change. Taking the first robotic arm 210 and instrument 310 on the first robotic arm 210 as an example, in response to the replacing of the imaging device 330, the controller 250 controls the wrist joint assembly 1030 of the first input device 102 to move, so that the orientation of the first input device 102 is aligned with the orientation of the end effector of the first instrument 310. In some embodiments, after replacing the imaging device 340 and the orientation of the imaging device 340 is locked, the controller 250 controls the motion of the wrist joint assembly 1030 to perform the alignment. In some embodiments, the response to the replacing the imaging device 340 can be triggered by the system detecting that the imaging device 330 has been mounted on the robotic arm.

In some embodiments, the instrument may need to be replaced during the surgery, for instance, switching from a bipolar electrocoagulation forceps instrument to a monopolar electrocoagulation knife instrument. After replacing the new instrument onto the robotic arm, the orientation of the end effector of the reloaded instrument is different from the orientation of the previous instrument. In response to the replacing the instrument, the controller 250 controls the wrist joint assembly 1030 of the input device 102 to move, so that the orientation of the input device 102 is aligned with the orientation of the replaced instrument. In some embodiments, the controller 250 controls the motion of the wrist joint assembly 1030 to perform the alignment only after the orientation of the instrument is locked.

After the orientation of the input device 102 is aligned with the orientation of the instrument 310, the surgeon activates the master-slave control, and there is no need to realign the orientation of the input device 102 with the orientation of the instrument 310, which improves surgical efficiency.

In some embodiments, the master console 100 includes an operating part, which comprises multiple sets of master joints, including at least one of arm joint, wrist joint, and self-rotation joint. Arm joint can also be referred to as position joint, while wrist joint and self-rotation joint can also be referred to as orientation joints.

The operating part can include a master arm 110, with arm joints provided within the master arm 110 to change the position and orientation of the master arm 110. The operating part can further include a master wrist, with wrist joints provided within the master wrist to control the position and orientation of the master wrist. Optionally, the first operating part can further include a pushable component, and moving the pushable component can change the position and orientation of the first operating part.

The operating part can further include a drive device, such as a motor, which can be provided with an encoder to achieve automatic positioning and other corresponding control functions. The drive device can include at least one of a position drive device, a self-rotation drive device, and a wrist joint drive device. It is understood that this classification of drive devices is based on functional perspectives, and their specific implementations do not necessarily correspond to specific drive devices; instead, how each function corresponds to specific drive device can be flexibly determined. Optionally, one drive device can correspond to one or more functions.

The operating part can further include a display device for the operator to observe the slave operating device 20.

The slave operating device 20 can include slave joints, and all joints in the slave operating device 20 are collectively referred to as slave joints. Both the robotic arm 210 and the instrument need to be provided with joints. In the embodiments according to the present disclosure, the joints in the robotic arm 210 are referred to as robotic arm slave joints, and the joints in the instrument are referred to as instrument slave joints. Optionally, the robotic arm 210 can further include at least one link connected by robotic arm slave joints. Optionally, the robotic arm 210 further includes multiple links connected via slave joints, and the robotic arm 210 is configured to adjust the spatial position of the instrument before or during the surgery.

The end effector includes a tissue grasper, a needle driver, scissors, a retractor, an electrosurgical cautery instrument, a stapler, a surgical clip applier, an ultrasonic cutter, a suction/irrigation instrument, a catheter, an ultrasonic probe, etc.. The imaging device can be an endoscope.

The slave operating device 20 can further include drive devices such as motors, which can drive the motion of the robotic arm 210 and the instrument, thereby changing the position and orientation of the end effector. Similarly, the drive device in the slave operating device 20 can include at least one of a position drive device, a self-rotation drive device, and a wrist joint drive device.

Specifically, a control mapping of position and orientation is established between the master arm 110, the master wrist and the instrument of the slave operating device 20. The mapping can correspond to position relationships, which can be proportional distances, corresponding distance trends, or other corresponding relationships. Alternatively, the mapping can correspond to motion relationships, which can be corresponding motion orientations, corresponding motion trends, etc. Thus, the operator can control the instrument to perform corresponding actions (such as pitch, yaw, roll, clamping, etc.) by operating the master arm 110 and the master wrist.

The surgical robot can include multiple master consoles 100 and/or multiple slave operating devices 20. The multiple master consoles 100 each can include multiple operating parts and the slave operating devices each can include multiple instruments, which are not limited in the embodiments according to the present disclosure. For instance, there can be two master arms 110, namely a lefthand arm and a right-hand arm corresponding to the left and right hands respectively, for easy operation by the surgeon's left and right hands. Optionally, there can be one or more than two master arm 110, which can be flexibly configured according to actual situations.

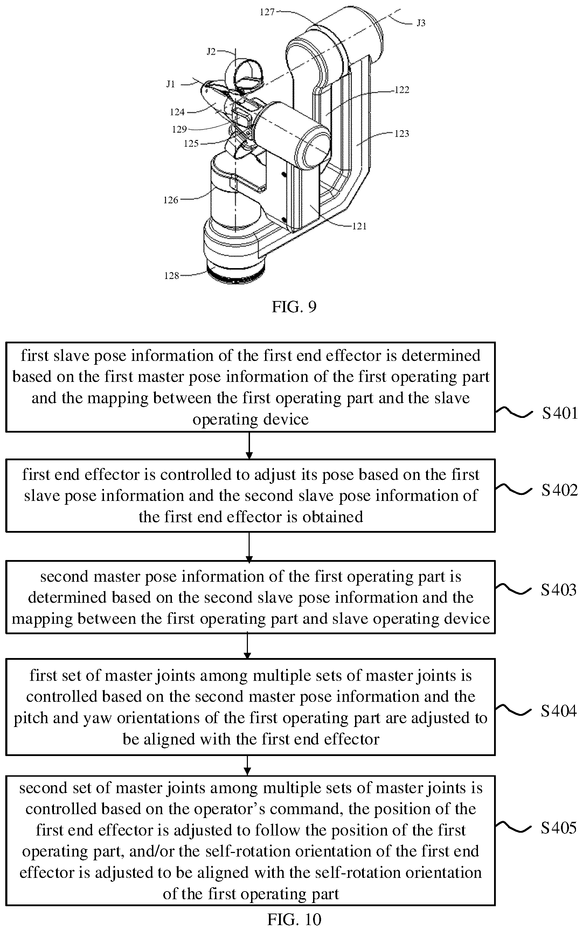

FIG. 9 illustrates a schematic diagram of the master wrist of an operating part according to an embodiment according to the present disclosure, showing that the master wrist has multiple degrees of freedom, generally at least three degrees of freedom. The master wrist comprises a first link 121, a second link 122, a third link 123, a gripper 124, a first rotation joint 125, a second rotation joint 126, a third rotation joint 127, a fourth rotation joint 128, and a pushable part 129. The gripper 124 is mounted to one end of the first link 121 via the first rotation joint 125. The pushable part includes two push buttons 129 disposed on the gripper 124. The other end of the first link 121 is mounted to one end of the second link 122 via the second rotation joint 126; the other end of the second link 122 is mounted to one end of the third link 123 via the third rotation joint 127; the other end of the third link 123 is mounted to the master hand arm 110 via the fourth rotation joint 128. The first rotation joint 125 is a self-rotation joint, while the second rotation joint 126, the third rotation joint 127, and the fourth rotation joint 128 are wrist joints.

The gripper 124 is rotatably connected to the first link 121 via the first rotation joint 125, allowing the gripper 124 to rotate about the first rotation axis J1 of the first rotation joint 125. The first link 121 is rotatably connected to the second link 122 via the second rotation joint 126, allowing the first link 121 to rotate about the second rotation axis J2 of the second rotation joint 126. The second link 122 is rotatably connected to the third link 123 via the third rotation joint 127, allowing the second link 122 to rotate about the third rotation axis J3 of the third rotation joint 127. The third link 123 is rotatably connected to the master hand arm 110 via the fourth rotation joint 128, allowing the third link 123 to rotate about the rotation axis of the fourth rotation joint 128.

In the illustrated embodiment, the rotation axes of the first rotation joint 125, the second rotation joint 126, and the third rotation joint 127-the first rotation axis J1, the second rotation axis J2, and the third rotation axis J3-intersect at one point. The master wrist adopts a design where multiple axes intersect at one point, resulting in relatively decoupled orientation and position of the entire master hand which facilitates kinematic calculations.

The surgical robot may further include a controller, which can be integrated into the master console or the slave operating device 20. Alternatively, the controller can be independent of the master console 100 and the slave operating device 20, for instance, deployed locally or in the cloud. The controller may consist of one or more processors. It should be noted that there can be multiple controllers, each handling different information. Among these multiple controllers, there can be a primary controller with the rest being secondary controllers, or they can all be relatively independent.

FIG. 10 is a schematic flowchart illustrating a control method for a surgical robot in an embodiment according to the present disclosure. The method is applied in a surgical robot comprising a master console and a slave operating device. The master console includes a first operating part, which comprises multiple sets of master joints. The slave operating device includes a first end effector. The method includes:

Step S401: the first slave pose information of the first end effector is determined based on the first master pose information of the first operating part and the mapping between the first operating part and the slave operating device.

After moving the first operating part from its initial position, the corresponding first master pose information is obtained according to the surgical needs of the operator. Specifically, the pose information of the first operating part can be obtained based on the pose information of specified point(s) of the first operating part. The number and position of thee specified point(s) can be configured according to needs. Optionally, the specified point(s) can be located in the distal region of the pushable part, for instance, at the intersection of the axes of the first rotation joint 125, the second rotation joint 126, and the third rotation joint 127. In other embodiments, the specified point(s) can be located in the central region of the pushable part. The number of specified point(s) can be one or more. The first master pose information can be obtained in real-time according to preset rules or only once when in a stationary state. The first master pose information can include position information and orientation angle information of the first operating part in the first coordinate based on at least one coordinate axis. The first coordinate can optionally be the coordinate of a display device.

Optionally, the first master pose information is determined based on the master joint variable corresponding to the motion of the first operating part from its initial position. For instance, variable, such as position information and/or rotation information, of each master joint in the first operating part is acquired via sensors, and the first master pose information is obtained based on the master joint variables. The sensors can include angle sensors that detect rotational motion of the joints and displacement sensors that detect linear motion of the joints, specifically configured according to the type of joint. Alternatively, the first master pose information can be obtained by a processor based on the master joint variables.

The first slave pose information can be the pose information of the first end effector in the base coordinate of the first end effector. The first slave pose information represents the expected pose that the slave operating device should follow due to the motion of the operating part.

In some embodiments according to the present disclosure, the first slave pose information of the first end effector is determined based on the first master pose information corresponding to the first operating part after motion and the mapping between the coordinate of the display device and the base coordinate of the first end effector.

Step S402: the first end effector is controlled to adjust its pose based on the first slave pose information and the second slave pose information of the first end effector is obtained.

Specially, the step S402 comprises:
Step S4021: the first end effector is controlled to adjust its pose based on the first slave pose information.

Specifically, the present step includes:
First, inversely resolving the first slave pose information of the first end effector to target variable, such as target position information and/or target rotation information, of the first slave joint of the first end effector.
Second, controlling the motion of the first slave joints based on the target variable of the first slave joint of the first end effector using kinematics principles. Optionally, operation commands for the drive device are determined based on the target variable of the first slave joint of the first end effector to control the motion of the slave operating device by the drive device. The first slave drive device may include a slave position drive device and a slave self-rotation drive device. The operation commands for the first slave drive device include the operation commands for the slave position drive device and for the slave self-rotation drive device. The robotic arm with movable joints and the first instrument are controlled through the operation commands for the slave position drive device and for the slave self-rotation drive device, so that the pose of the first end effector is adjusted to approach (or reach) the target variable of the first slave joint of the first end effector to follow follows the motion of the first operating part.

Optionally, the first slave drive device may further include a wrist joint drive device. When the first instrument does not have an instrument wrist joint, the wrist joint drive device cannot function. In this case, even if the wrist joint drive device is operated, it cannot drive the wrist joint motion of the first instrument. Therefore, the wrist joint drive device may remain stationary, specifically including:
Transmitting an operation command for the wrist joint drive device, which instructs the wrist joint drive device to remain stationary;
Or not transmitting an operation command for the wrist joint drive device.

Similarly, if the first instrument does not have a self-rotation joint, the slave self-rotation drive device cannot function. In this case, even if the slave rotation drive device is operated, it cannot drive the self-rotation motion of the first instrument. Therefore, the slave rotation drive device can remain stationary.

The first slave drive devices including the slave position drive device, slave rotation drive device, and wrist joint drive device, are divided based on functionality. The specific implementation of the first slave drive device does not necessarily correspond to specific drive devices, and the correspondence between each function and specific drive devices can be flexibly configured. Optionally, one drive device may correspond to one or more functions.

Step S4022: the second slave pose information of the first end effector is obtained.

Specifically, the present step includes:
Acquiring second slave pose information based on specified point(s) in the first end effector. Optionally, the specified point(s) is located in the distal region of the first end effector, such as at the head of the first end effector. In other embodiments, the specified point(s) may be located in other regions of the first end effector, such as in the central region. The number of the specified point(s) may be one or more. The second slave pose information can be acquired in real-time according to preset rules or acquired only once when in a stationary state. The second slave pose information can be the pose information of the first end effector in the base coordinate of the first end effector, specifically including:
Acquiring the first slave joint variable, such as pose information and/or rotation information, corresponding to the adjusted pose of the first end effector; determining the second slave pose information based on the first slave joint variable.

Optionally, the pose information of the first end effector can be determined by joint variable in the robotic arm and the first instrument, such as actual position and orientation angle information. The pose information of the first end effector is obtained by acquiring the current pose information and/or rotation information of the robotic arm slave joints and the first instrument slave joints after motion and resolving the current pose information and/or rotation information of the robotic arm slave joints and the first instrument slave joints. Similarly, the first slave joint variable can be acquired through sensors and the pose information of the first end effector can be acquired based on the first slave joint variable. Alternatively, the pose information of the first end effector can be acquired by a processor based on the first slave joint variable.

In some embodiments of the disclosure, the target pose of the first end effector in the slave operating device is calculated by converting the first master pose information of the first operating part after controlled motion through coordinate transformation. The target pose is resolved into target variable of the first slave joint. The pose of the first end effector is adjusted by controlling the motion of the first slave joints to make it approach (or reach) the target variable of the first slave joint. Furthermore, the second slave pose information is determined by resolving the actual spatial parameters of the first slave joint corresponding to the adjusted pose of the first end effector. Thus, the second slave pose information of the first end effector is determined based on the actual pose of the first end effector after motion, which ensures the authenticity and reliability of the pose information and further includes rotation data in the pose information due to the motion of the first end effector, laying the foundation for further determining the motion of the first operating part following the first end effector.

Step S403: the second master pose information of the first operating part is determined based on the second slave pose information and the mapping between the first operating part and the slave operating device.

Specifically, the present step includes the following sub-steps:
Step S4031: the third slave pose information of the first end effector in the coordinate of the imaging device is determined based on the second slave pose information and the mapping between the base coordinate of the first end effector and the coordinate of the imaging device.
Step S4032: the third master pose information of the end of the first operating part in the coordinate of the display device is determined based on the third slave pose information and the mapping between the coordinate of the imaging device and the coordinate of the display device.
Step S4033: the fourth master pose information of the end of the first operating part in the base coordinate of the master console is determined based on the third master pose information and the mapping between the coordinate of the display device and the base coordinate of the master console.
Step S4034: the fourth master pose information is used as the second master pose information.

The present embodiment of the disclosure converts the second slave pose information of the first end effector in the base coordinate of the first end effector into the second master pose information of the end of the first operating part in the base coordinate of the master console by coordinate transformation.

Step S404: the first set of master joints among multiple sets of master joints is controlled based on the second master pose information and the pitch and yaw orientations of the first operating part are adjusted to be aligned with the first end effector.

If the first set of master joints is a wrist joint, adjustments to the wrist joint can affect the pitch and yaw orientations. Therefore, the pitch and yaw orientations of the first operating part can be aligned with the first end effector by adjusting the wrist joint. The present step specifically includes steps S4041 to S4043:
Step S4041: the second master pose information is inversely solved into variables of the first set of master joints, such as target position and/or target rotation information.

Based on kinematics, the second master pose information is inversely solved into the joint variable corresponding to the first set of master joints, such as target position and/or target rotation information. Optionally, the second master pose information can be inversely solved into spatial parameter information of all joints, or only into joint variable of the wrist joint that needs to be adjusted in the next step based on the actual situation. It is understandable that inversely solving only the variable of the wrist joint can improve processing efficiency and save system resources.

Step S4042: the control command for the drive device is determined based on the variables of the first set of master joints. The control command for the drive device includes a control command for the first set of master joints, which at this time includes a control command for the wrist joint.

In the aforementioned step, if only the variable of the wrist joint is inversely solved, the control command for the drive device in the present step only includes the control command for the wrist joint. In the aforementioned step, if the variables of all joints are inversely solved, such as including position joint variable and/or self-rotation joint variable, then the control command for the drive device can correspondingly include control command(s) for position joint and/or self-rotation joint. It is understandable that even if the variables of all joints are inversely solved in the aforementioned step, the control command for the drive device can only include the control command for the wrist joint.

Step S4043: Control the wrist joint is controlled based on the control command for the wrist joint, and the pitch and yaw orientations of the first operating part are adjusted to be aligned with the first end effector.

When the first instrument of the slave operating device does not have an instrument wrist joint, and the first operating part has a wrist joint, after the slave operating device follows the motion of the first operating part, since the robotic arm reaches the target position through rotation, the pose will be rotated relative to the coordinate of the imaging device after reaching the target position. The rotation causes change in the orientation data of the pose data, i.e., change in orientation. Only the master wrist joint can control the rotation. Therefore, the wrist joint of the first operating part is moved to an appropriate pose by calculating the master hand, which can make the operator's orientation feel consistent with the first end effector, thereby enhancing the intuitive operation experience. Optionally, the master wrist of the first operating part, especially the wrist joint of the master wrist is adjusted.

Step S405: the second set of master joints among multiple sets of master joints is controlled based on the operator's command, the position of the first end effector is adjusted to follow the position of the first operating part, and/or the self-rotation orientation of the first end effector is adjusted to be aligned with the self-rotation orientation of the first operating part.

The second set of master joints includes at least one of position joint, wrist joint, and self-rotation joint, but the second set of master joints is different from the first set of master joints. Therefore, when the first set of master joints is a wrist joint, the second set of master joints cannot be a wrist joint.

Optionally, the second set of master joints is a position joint. According to the operator's command, the position of the first operating part can be changed by moving the position joint. Alternatively, the second set of master joints is a self-rotation joint. According to the operator's command, the self-rotation orientation of the first operating part can be changed by rotating the self-rotation joint. Alternatively, the first operating part includes a position joint and a self-rotation joint. According to the operator's command, the position of the first operating part can be changed by moving the position joint, and the self-rotation orientation of the first operating part can be changed by rotating the self-rotation joint.

If the operator's command causes the position of the first operating part to change, then the position of the first end effector is controlled to follow the position of the first operating part. Thus, by controlling the first set of master joints based on the second master pose information and controlling the second set of master joints based on the operator's command, the position of the first end effector follows the position of the first operating part, and the orientation of the first end effector is aligned with the orientation of the first operating part.

If the self-rotation orientation of the first operating part is changed by the operator's command, the self-rotation orientation of the first end effector is controlled to be aligned with the self-rotation orientation of the first operating part. Thus, by controlling the first set of master joints based on the second master pose information and controlling the second set of master joints based on the operator's command, the orientation of the first end effector is made to be aligned with the orientation of the first operating part.

If both position and self-rotation orientation of the first operating part are changed by the operator's command, the position of the first end effector is controlled to follow the position of the first operating part, and the self-rotation orientation of the first end effector is controlled to be aligned with the self-rotation orientation of the first operating part. Thus, by controlling the first set of master joints based on the second master pose information and controlling the second set of master joints based on the operator's command, the position of the first end effector is made to follow the position of the first operating part, and the orientation of the first end effector is made to be aligned with the orientation of the first operating part.

In some embodiments according to the present disclosure, the pitch and yaw orientations of the first operating part are adjusted to be aligned with the first end effector by controlling the first set of master joints based on the second master pose information; and the position of the first end effector is adjusted to follow the position of the first operating part, and/or the self-rotation orientation of the first end effector is adjusted to be aligned with the self-rotation orientation of the first operating part by controlling the second set of master joints among multiple sets of master joints based on the operator's command, which is more conducive to the operator's intuitive operation experience.

In the control method for a surgical robot provided in another embodiment according to the present disclosure, the surgical robot includes a master console and a slave operating device. The master console includes a first operating part, which comprises multiple sets of master joints. The slave operating device includes a first instrument with a first end effector. The first set of master joints is a position joint. Specifically, the method includes:
Determining the first slave pose information of the first end effector based on the first master pose information of the first operating part, according to the mapping between the first operating part and the slave operating device; controlling the first end effector to adjust its pose based on the first slave pose information, and acquiring the second slave pose information of the first end effector; determining the second master pose information of the first operating part based on the second slave pose information, according to the mapping between the first operating part and the slave operating device. The implementation principle is the same as the aforementioned steps and will not be repeated here.

The controlling the first set of master joints among the multiple sets of master joints based on the second master pose information to adjust the position of the first operating part to follow the position of the first end effector, are divided into three steps:
Step 1: Inversely resolving the second master pose information into position joint variable, such as target position and/or target rotation information. Similarly, it is also possible to simultaneously inversely resolve into joint variable of all joints, including wrist joint variable and self-rotation joint variable. It is understood that inversely resolving only position joint variable can improve processing efficiency and save system resources.
Step 2: Determining the control command for drive device based on the position joint variable, where the control command for drive device includes control command for the first set of master joints, which is control command for position joint at this time. In the aforementioned steps, if all joint variables are inversely resolved, the control command for drive device may further include control command(s) for wrist joint and/or self-rotation joint. Of course, even if all joint variables are inversely resolved in the aforementioned steps, the control command for drive device may only include control command for position joint.
Step 3: Controlling the position joint according to the control command for position joint to adjust the position of the first operating part to follow the position of the first end effector.

The second set of master joints among the multiple sets of master joints is controlled according to the operator's command to adjust the orientation of the first operating part to be aligned with the orientation of the first end effector, which is explained in three situations:
Situation 1: If the second set of master joints is wrist joint, the position joint is controlled according to the second master pose information to adjust the position of the first operating part to follow the position of the first end effector; the wrist joint is controlled according to the operator's command to adjust the pitch and yaw orientations of the first end effector to be aligned with the pitch and yaw orientations of the first operating part.
Situation 2: If the second set of master joints is self-rotation joint, the position joint is controlled according to the second master pose information to adjust the position of the first operating part to follow the position of the first end effector; the self-rotation joint is controlled according to the operator's command to adjust the self-rotation orientation of the first end effector to be aligned with the self-rotation orientation of the first operating part.
Situation 3: If the second set of master joints includes both wrist joint and self-rotation joint, the position joint is controlled according to the second master pose information to adjust the position of the first operating part to follow the position of the first end effector; the wrist joint and self-rotation joint is controlled according to the operator's command to adjust the self-rotation orientation of the first end effector to be aligned with the self-rotation orientation of the first operating part.

In some embodiments according to the present disclosure, the position of the first operating part is adjusted to follow the first end effector by controlling the first set of master joints among the multiple sets of master joints according to the second master pose information, and the orientation of the first end effector is adjusted to be aligned with the orientation of the first operating part by controlling the second set of master joints among the multiple sets of master joints according to the operator's command, which are more conducive to the operator's intuitive operational experience.

In the control method for a surgical robot provided in another embodiment according to the present disclosure, the surgical robot includes a master console and a slave operating device. The master console includes a first operating part, which comprises multiple sets of master joints. The slave operating device includes a first instrument with a first end effector. The first set of master joints is self-rotation joint. Specifically, the method includes:
Determining the first slave pose information of the first end effector based on the first master pose information of the first operating part, according to the mapping between the first operating part and the slave operating device; controlling the first end effector to adjust its pose based on the first slave pose information, and acquiring the second slave pose information of the first end effector; determining the second master pose information of the first operating part based on the second slave pose information, according to the mapping between the first operating part and the slave operating device; the implementation principle is the same as the aforementioned steps and will not be repeated here.

The first set of master joints among the multiple sets of master joints is controlled based on the second master pose information to adjust the self-rotation orientation of the first operating part to be aligned with the self-rotation orientation of the first end effector. When the first set of master joints is self-rotation joint, the adjustment of the self-rotation joint can affect the self-rotation orientation. Therefore, the self-rotation orientation of the first operating part can be aligned with that of the first end effector by adjusting the self-rotation joint, which specifically includes three steps:
Step 1: Inversely resolving the second master pose information into self-rotation joint variable, such as target position and/or target rotation information. Similarly, it is also possible to inversely resolve the second master pose information into joint variables of all joints, including wrist joint variable and position joint variable. It is understood that inversely resolving only self-rotation joint variable can improve processing efficiency and save system resources.
Step 2: Determining the control command for drive device based on the self-rotation joint variable, where the control command for drive device includes control command for the first set of master joints, which is control command for self-rotation joint. In the aforementioned step, if all joint variables are inversely resolved, the control command for drive device may further include control command(s) for wrist joint and/or position joint. Of course, even if all joint variables are inversely resolved in the aforementioned steps, the control command for drive device may only include control command for self-rotation joint.
Step 3: Controlling the wrist joint according to the control command for self-rotation joint to adjust the self-rotation orientation of the first operating part to be aligned with the self-rotation orientation of the first end effector.

The position of the first end effector is adjusted to follow the first operating part, and/or the pitch and yaw orientations of the first end effector is adjusted to be aligned with the first operating part by controlling the second set of master joints among multiple sets of master joints according to the operator's command, which is divided into three scenarios:

### Scenario 1:

If the second set of master joints is a wrist joint, the self-rotation joint is controlled based on the second master pose information to adjust the self-rotation orientation of the first operating part to be aligned with the self-rotation orientation of the first end effector; the wrist joint is controlled according to the operator's command to adjust the pitch and yaw orientations of the first end effector to be aligned with the pitch and yaw orientations of the first operating part.

### Scenario 2:

If the second set of master joints is a position joint, the self-rotation joint is controlled based on the second master pose information to adjust the self-rotation orientation of the first operating part to be aligned with the self-rotation orientation of the first end effector; the position joint is controlled according to the operator's command to adjust the position of the first end effector to follow the position of the first operating part.

### Scenario 3:

If the second set of master joints includes both wrist joint and position joint, the self-rotation joint is controlled based on the second master pose information to adjust the self-rotation orientation of the first operating part to be aligned with the self-rotation orientation of the first end effector; the wrist joint and position joint are controlled according to the operator's command to adjust the position of the first end effector to follow the position of the first operating part, and adjust the pitch and yaw orientations of the first end effector to be aligned with the pitch and yaw orientations of the first operating part.

In the embodiments according to the present disclosure, the self-rotation orientation of the first operating part is adjusted to be aligned with the self-rotation orientation of the first end effector by controlling the first set of master joints among multiple sets of master joints based on the second master pose information; the position of the first end effector is adjusted to follow the position of the first operating part, and/or the pitch and yaw orientations of the first end effector are adjusted to be aligned with the first operating part by controlling the second set of master joints among multiple sets of master joints according to the operator's command, ultimately achieving alignment between the orientation of the first end effector and the first operating part, which is more conducive to the operator's intuitive experience.

In another embodiment according to the present disclosure, to endow the second set of master joints with autonomous mobility, the step of controlling the second set of master joints among multiple sets of master joints according to the operator's command includes:
Controlling the second set of master joints among multiple sets of master joints according to the operator's command, and the change in the second set of master joints does not follow the change in the first end effector.

When it is necessary to adjust the first operating part according to the first end effector by adjusting the first set of master joints, to endow the second set of master joints with autonomous mobility, it is not necessary to control the motion of the second set of master joints based on the variables of the first set of joints. Therefore, the control command for the first set of master joints does not need to affect the second set of master joints, so they do not need to be sent to the second set of master joints. Alternatively, even if the control command for the first set of master joints is sent to the second set of master joints, the second set of master joints does not respond. Additionally, control command for other joint different from the first set of master joints do not need to take effect. Similarly, control command for other joint can be generated, not generated, or generated but not further sent or responded to. That is, the joint variable derived from the inverse solution based on the second master pose information, as well as the control command for joint generated based on these variables, do not need to affect the second set of master joint.

For instance, when the first set of master joints is a wrist joint, the control commands for the wrist joint, position joint, and self-rotation joint do not need to affect the second set of master joints. Therefore, the control command for wrist joint does not need to be sent to the second set of master joints. Alternatively, even if the control command for wrist joint is sent to the second set of master joints, the second set of master joints does not respond. Meanwhile, the change in other joints of the first operating part, including the second set of master joints of the first operating part, does not need to follow the change in the first end effector. Optionally, the aforementioned control command for position joint does not need to take effect, so it can be generated but do not need to be sent to the second set of master joints, or it can simply not be generated. The aforementioned control command for self-rotation joint does not need to take effect, so it can be generated but does not need to be sent to the second set of master joints, or it can simply not be generated.

In the embodiment, the first set of master joints among multiple sets of master joints is controlled based on the second master pose information and the second set of master joints among multiple sets of master joints is controlled according to the operator's command. The change in the second set of master joints does not follow the change in the first end effector, which not only promptly adjusts the first set of master joints of the first operating part, being more conducive to the operator's intuitive operational experience, but also ensures the autonomous mobility of the second set of master joints, thereby facilitating the acceptance of the operator's command.

In another embodiment according to the present disclosure, to prevent the first end effector of the slave operating device from following the motion due to the motion of the first set of master joints, the change in the first end effector does not follow the change in the first set of master joints, specifically including:
When the first set of master joints is a wrist joint, to prevent the first end effector of the slave operating device from following the motion due to the adjustment of the wrist joint, it is also necessary to ensure that the position and orientation of the first end effector are not adjusted based on the motion of the first set of master joints. For instance, the control command for wrist joint is not sent to the driving device of the first end effector, or even if the command is sent to the driving device of the first end effector, the driving device does not respond to the command.

When the first set of master joints is a position joint, to prevent the first end effector of the slave operating device from following the motion due to the adjustment of the position joint, it is also necessary to ensure that the control command for position joint is not sent to the driving device of the first end effector, or even if the command is sent to the driving device of the first end effector, the driving device does not respond to the command.

When the first set of master joints is a self-rotation joint, to prevent the first end effector of the slave operating device from following the motion due to the adjustment of the self-rotation joint, it is also necessary to ensure that the control command for self-rotation joint is not sent to the driving device of the first end effector, or even if the command is sent to the driving device of the first end effector, the driving device does not respond to the command.

In the embodiments according to the present disclosure, the adjustment of the orientation of the first operating part following the motion of the slave operating device is achieved by controlling the motion of the first set of master joints, while the change in the first end effector does not follow the change in the first set of master joints, which promptly adjusts the master joint variable of the first operating part, being more conducive to the operator's intuitive operational experience, and simultaneously ensures that the motion of the first set of master joints does not continue to trigger the slave operating device to follow, thereby achieving normal surgical control by the operator.

In another embodiment according to the present disclosure, the master console of the surgical robot may further include a second operating part, which comprises multiple sets of master joints, such as a third set of master joints. The slave operating device may further include a mechanical part, a second slave joint, and a second instrument. The second instrument includes a second end effector.

Unlike the first operating part, which adjusts its orientation based on the motion of the slave operating device, the second operating part does not adjust its orientation based on the motion of the slave operating device. Specifically, the method includes:
Step 1: Determining the fifth slave pose information of the second end effector based on the third master pose information of the second operating part and the mapping between the second operating part and the slave operating device.

The fifth pose information of the second end effector in the base coordinate of the second end effector is determined based on the third pose information of the second operating part in the coordinate of the display device and the mapping between the coordinate of the display device and the base coordinate of the second end effector.

The present step is the same as step S401. Based on the surgical needs of the operator, the second operating part can be moved from its initial position, and the slave operating device will follow this motion. Specifically, when determining the fifth slave pose information of the second end effector in its base coordinate, the pose information of the end of the second operating part can be obtained based on the pose information of specified point(s) in the second operating part. The number and positions of the specified point(s) can be configured as needed. Optionally, the specified point(s) are located in the distal region of the pushable part, for instance, at the end of the pushable part. In other embodiments, the specified point(s) can be located in the central region of the pushable part. The number of specified point(s) can be one or more. The fifth slave pose information can be obtained in real-time according to preset rules or only obtained once when in a stationary state. The third master pose information of the end of the second operating part can include pose information and orientation angle information based on at least one coordinate axis in the first coordinate where the second operating part is located. Optionally, the first coordinate is the coordinate of the display device.

Optionally, the third master pose information of the end of the second operating part is determined based on the joint variable of the corresponding master joints after the motion of the second operating part. For instance, sensors are used to obtain the joint variable of each master joints in the second operating part, such as pose information and/or rotation information, and the third master pose information of the second operating part is obtained based on the joint variables. The sensors can include angle sensors that sense rotational motion of the joints and displacement sensors that sense linear motion of the joints specifically configured according to the type of joints. Alternatively, a processor is used to obtain the third master pose information of the second operating part based on the joint variable.

Step 2: Controlling the second end effector to adjust its pose based on the fifth slave pose information without obtaining the sixth slave pose information of the second end effector; or obtaining the sixth slave pose information of the second end effector without controlling the third set of master joints in the multiple sets of master joints of the second operating part based on the sixth slave pose information.

First, the fifth slave pose information of the second end effector is inversely solved to obtain the target variable of the second slave joint of the second end effector, such as target position and/or target rotation.

Then, based on kinematics, the motion of the second slave joint is determined according the target variables of the second slave joint. Optionally, the motion of the slave operating device is controlled through the second slave drive device. For instance, the motion of a robotic arm with movable joints and the second instrument is controlled so that the second end effector moves to a second updated pos which is close to (or reaches) the target variable of the second slave joint.

Optionally, the drive device includes a position drive device and a rotation drive device, and the operation commands for the drive device include operation commands for the position drive device and operation commands for the rotation drive device. The motion of the slave operating device is controlled through the operation command for the position drive device and the operation command for the rotation drive device, so that the second end effector adjusts its pose.

Optionally, the drive device further includes a wrist joint drive device. When the second instrument has an instrument wrist joint, after the second end effector adjusts its pose, the second operating part does not adjust its orientation based on the motion of the slave operating device, thus the sixth slave pose information corresponding to the adjusted pose of the second end effector is not obtained. Alternatively, the sixth slave pose information is obtained but is not used to control the motion of the third set of master joints of the second operating part.

Step 3: Controlling the fourth set of master joints in the second operating part based on the operator's command, adjusting the position of the second end effector to follow the position of the second operating part, and adjusting the orientation of the second operating part to be aligned with the orientation of the second end effector.

For instance, the first instrument is equipped with an ultrasonic scalpel without a wrist joint, and the second instrument is equipped with a grasping forceps with a wrist joint, so the degrees of freedom of motion of the grasping forceps and the ultrasonic scalpel are different.

For easier understanding, we can refer to the master arm of the first operating part as the left arm, the master wrist of the first operating part as the left wrist, the master arm of the second operating part as the right arm, the master wrist of the second operating part as the right wrist, and the first set of master joints as wrist joint. The left hand controls the ultrasonic scalpel, and the right hand controls the grasping forceps. Correspondingly, the method includes:
Controlling the motion of the wrist joint of the left wrist, which means the left hand adopts the mode in which the first operating part is adjusted based on the motion of the slave operating device in the embodiments according to the present disclosure;
Not controlling the motion of the wrist joint of the right wrist, which means the right hand adopts the mode in which the second operating part is not adjusted based on the motion of the slave operating device in the embodiments according to the present disclosure.

In the embodiments according to the present disclosure, the adjustment of the first operating part based on the motion of the slave operating device is achieved by providing the first operating part and the second operating part in the master console, which is beneficial to the intuitive operational experience of the operator. The second operating part is not adjusted based on the motion of the slave operating device, thus different adjustment modes of the first operating part and the second operating part can be realized, flexibly adapting to various types of instruments.

In another embodiment according to the present disclosure, apart from achieving the motion of the first operating part based on the slave operating device by adjusting only one type of the multiple sets of master joints as described above, the multiple sets of master joints can further include a fifth set of master joints. The adjustment of the first operating part based on the slave operating device is achieved by adjusting two types of joints among the multiple sets of master joints, namely the first set of master joints and the fifth set of master joints. After controlling the first set of master joints among the multiple sets of master joints based on the second master pose information, the method further includes:
Controlling the fifth set of master joints among the multiple sets of master joints based on the second master pose information, which specifically includes:
Inversely resolving the second master pose information into variable of the fifth set of master joints, such as target position and/or target rotation. The specific implementation principle is the same as inversely resolving the second master pose information into variable of the first set of master joints, and will not be repeated here.

Determining the control command for drive device based on the variable of the fifth set of master joints. The control command for drive devices includes control command for the fifth set of master joints. The specific implementation principle is the same as determining the control command for drive device based on the variable of the first set of master joints, and will not be repeated here.

Controlling the fifth set of master joints based on the control command for the fifth set of master joints. The specific implementation principle is the same as controlling the first set of master joints based on the control command for the first set of master joints, and will not be repeated here.

When two types of joints are needed, control commands for at least two corresponding joints: control command for the first set of master joints and control command for the fifth set of master joint, are required.

For instance, the first set of master joints is a wrist joint, and the fifth set of master joints is a self-rotation joint. When adjusting the first operating part to follow the adjustment of the slave operating device through the wrist joint and the self-rotation joint, it is necessary to inversely resolve the second master pose information into variable of the wrist joint and variable of the self-rotation joint, and generate control command for the wrist joint and control command for the self-rotation joint based on the variable of the wrist joint and the variable of the self-rotation joint respectively. The wrist joint is controlled based on the control command for the wrist joint, and the self-rotation joint is controlled based on the control command for the self-rotation joint, jointly adjusting the first operating part to follow the adjustments of the slave operating device. The present embodiment does not provide examples for all combinations.

In the present embodiment according to the present disclosure, the adjustment of the first operating part based on the motion of the slave operating device is achieved by controlling two types of joints among the multiple sets of master joints to move, which is more conducive to the intuitive operational experience of the operator.

In another embodiment according to the present disclosure, the multiple sets of master joints include a first set of master joints and a fifth set of master joints. The adjustment of the first operating part based on the slave operating device is achieved by adjusting two types of joints among the multiple sets of master joints, namely the first set of master joints and the fifth set of master joints. To endow the second set of master joints with autonomous mobility, the controlling the second set of master joints among the multiple sets of master joints based on the operator's command includes:
Controlling the second set of master joints among the multiple sets of master joints based on the operator's command, and the change in the second set of master joints not following the change in the first end effector.

When it is necessary to adjust the first operating part by adjusting the two types of joints, namely the first set of master joints and the fifth set of master joints, it is not necessary to control the motion of the second set of master joints based on the joint variables corresponding to these two types of joints. The control command for the first set of master joints and the control command for the fifth set of master joints are not sent to the second set of master joints, or even if they are sent, the second set of master joints does not respond. Additionally, control command for joint different from those for the first set of master joints and the second set of master joints does not need to take effect. Similarly, it can be generated, not generated, or generated but not further sent or responded to.

For instance, the first set of master joints is a wrist joint, and the fifth set of master joints is a self-rotation joint. The control command for the wrist joint, the control command for the self-rotation joint, and the control command for the position joint do not need to take effect on the second set of master joints. The control command for the wrist joint and the control command for the self-rotation joint can be not sent to the second set of master joints, or sent but not responded to by the second set of master joints. The control command for the position joint can be not generated, or generated but not sent to the second set of master joints, or sent but not responded to by the second set of master joints. The present embodiment does not provide examples for all combinations.

In the present embodiment according to the present disclosure, the adjustment of the first operating part following the motion of the slave operating device is achieved by controlling two types of master joints among the multiple sets of master joints to move based on the second slave pose information, and the change in the second set of master joints does not follow the change in the first end effector, which only timely adjusts the master joint variable of the first operating part, being conducive to the intuitive operational experience of the operator, but also ensures the autonomous motion of the second set of master joints, thereby facilitating the acceptance of the operator's command.

In another embodiment according to the present disclosure, the multiple sets of master joints include a first set of master joints and a fifth set of master joints. To prevent the motion of the fifth set of master joints from causing the first end effector of the slave operating device to follow the motion, after the change in the first end effector does not following the change in the first set of master joint the method can further include:
The change in the first end effector does not following the change in the fifth set of master joint.

The specific implementation principle is the same as the principle for achieving that the change in the first end effector does not follow the change in the first set of master joints, and will not be repeated here.

In another embodiment according to the present disclosure, the operating part of the surgical robot can include not only the first operating part and the second operating part, but also a larger number of operating parts. The number of operating parts can be flexibly configured according to the actual surgical situation. The number of instruments of the slave operating device can be flexibly configured according to the actual surgical situation, and the end effectors mounted on each instrument can have different degrees of freedom of motion. In this control scenario, each operating part can freely choose the mode where the adjustment of first operating part follows the motion of the slave operating device as described above, or the mode where the orientation adjustment of second operating part does not follow the motion of the slave operating device as described above, thereby flexibly adapting to various types of instruments and meeting the needs of complex and personalized surgeries.

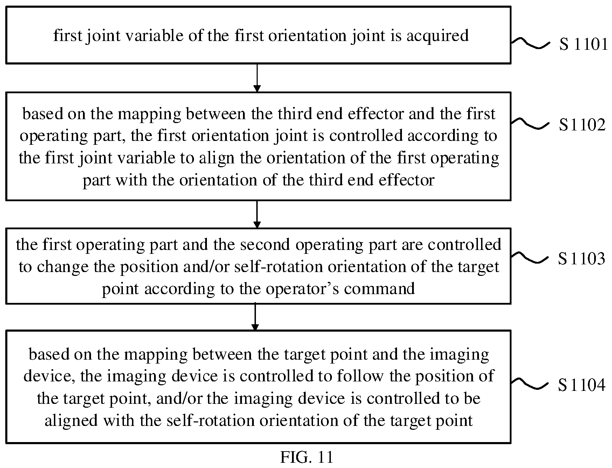

Referring to FIG. 11, in some embodiments, a schematic flowchart of a control method for a surgical robot according to an embodiment according to the present disclosure is provided. The surgical robot includes a master console and a slave operating device. The master console includes a first operating part and a second operating part, with a target point configured between the first operating part and the second operating part. The first operating part includes a first orientation joint, and the slave operating device includes a third end effector and an imaging device. Specifically, the method includes the following steps.

Step S1101: The first operating part includes a first orientation joint, and a first joint variable of the first orientation joint is acquired. The first orientation joint can be a wrist joint or a self-rotation joint, or may include both a wrist joint and a self-rotation joint.

Step S1102: Based on the mapping between the third end effector and the first operating part, the first orientation joint is controlled according to the first joint variable to align the orientation of the first operating part with the orientation of the third end effector. Since the first orientation joint directly affects the orientation of the first operating part, controlling the first orientation joint can align the orientation of the first operating part with the orientation of the third end effector.

Step S1103: The first operating part and the second operating part are controlled to change the position and/or self-rotation orientation of the target point according to the operator's command.

The position or self-rotation orientation of the target point, or both, can be changed according to the operator's surgical needs.

Step S1104: Based on the mapping between the target point and the imaging device, the imaging device is controlled to follow the position of the target point, and/or the imaging device is controlled to be aligned with the self-rotation orientation of the target point.

In the embodiments according to the present disclosure, by controlling the first orientation joint based on the first joint variable to align the orientation of the first operating part with the orientation of the third end effector, and by controlling the imaging device to follow the position of the target point and be aligned with the self-rotation orientation of the target point according to the operator's command, the orientation of the first operating part remains aligned with the orientation of the third end effector while the imaging device is being controlled by the operating part.

The control method for the surgical robot provided in the embodiments according to the present disclosure involves a surgical robot comprising a master console and a slave operating device. The master console includes a first operating part and a second operating part, with a target point configured between them. The first operating part includes a first orientation joint, and the slave operating device includes a third end effector and an imaging device. Specifically, the method includes following steps.

Step S1101: The first operating part includes a first orientation joint, and a first joint variable of the first orientation joint is acquired.

The present step includes two implementation methods:
First implementation method: Calculation and determination, including following sub-steps.

Step S 11011: fourth slave pose information of the third end effector is acquired.

The acquiring fourth slave pose information of the third end effector may comprise acquiring the fourth slave pose information based on specified point(s) in the third end effector. Optionally, the specified point(s) are located in the distal region of the third end effector, such as at the head of the third end effector. In other embodiments, the specified point(s) may be located in other regions of the third end effector, such as in the middle region. The number of specified point(s) can be one or more. The fourth slave pose information can be acquired in real-time according to preset rules or only once when in a stationary state. The fourth slave pose information can be the pose information of the third end effector in the base coordinate of the third end effector.

Specifically, the acquiring fourth slave pose information of the third end effector may comprise: acquiring a first slave joint variable, such as pose information and/or rotation information, corresponding to the third end effector; determining the fourth slave pose information based on the first slave joint variable.

Optionally, the pose information corresponding to the third end effector is determined through joint variable, such as actual position and orientation angle information, in the robotic arm and the third surgical instrument. That is, current joint variables, such as pose information and/or rotation information, of the robotic arm slave joint and the third surgical instrument slave joint in the slave operating device is acquired and solved into the fourth slave pose information of the third end effector. Similarly, the first slave joint variable can be acquired through sensors, and the fourth slave pose information of the third end effector can be acquired based on the first slave joint variable. For instance, the fourth slave pose information of the third end effector is acquired by a processor based on the first slave joint variable.

In the embodiments according to the present disclosure, the fourth slave pose information is determined by positively solving the actual joint variable of the first slave joint of the third end effector, thereby determining the fourth slave pose information of the third end effector based on the actual pose of the third end effector, which ensures the authenticity and reliability of the pose information.

Step S11012: fifth master pose information of the first operating part is determined based on the fourth slave pose information and the mapping between the first operating part and the slave operating device, specifically including the following steps.

Step 1: the fifth slave pose information of the third end effector in the coordinate of the imaging device is determined based on the fourth slave pose information and the mapping between the base coordinate of the third end effector and the coordinate of the imaging device.

Step 2: sixth master pose information of the first operating part end in the coordinate of the display device is determined based on the fifth slave pose information and the mapping between the coordinate of the imaging device and the coordinate of the display device.

Step 3: seventh master pose information of the first operating part end in the base coordinate of the master console is determined based on the sixth master pose information and the mapping between the coordinate of the display device and the base coordinate of the master console.

Step 4: the seventh master pose information is used as the fifth master pose information.

In the embodiments according to the present disclosure, the fourth slave pose information of the third end effector in the base coordinate of the third end effector is converted into the fifth master pose information of the first operating part end in the base coordinate of the master console through coordinate transformation.

Step S11013: the first joint variable is determined based on the fifth master pose information.

Based on kinematics, the fifth master pose information is inversely solved into the first joint variable, such as position and/or rotation information, corresponding to the first orientation joint of the first operating part. Optionally, only the first joint variable corresponding to the first orientation joint that needs to be adjusted in the next step can be inversely solved based on the actual situation, or the joint variable of all joints of the first operating part can be solved. For instance, if the first operating part further includes a first position joint and a first self-rotation joint, the joint variables corresponding to the first position joint and the first self-rotation joint can be inversely solved while inversely solving the first joint variable. It is understood that inversely solving only the first joint variable can improve processing efficiency and save system resources.

Second Implementation Method: Direct Acquisition.

Optionally, the first joint variable, such as pose information and/or rotation information, in the first operating part are acquired through sensors, and the fifth master pose information is obtained based on the master joint variable. Sensors may include angle sensors that sense joint rotational motion and displacement sensors that sense joint linear motion, specifically configured according to the type of joint. For instance, joint variable can be obtained by acquiring signals from encoders provided in the driving device. Similarly, joint variable of all joints in the first operating part can be obtained according to the actual situation.

Step S1102: Based on the mapping between the third end effector and the first operating part, the first orientation joint is controlled according to the first joint variable to align the orientation of the first operating part with the orientation of the third end effector. The first orientation joint may include a wrist joint and a self-rotation joint. The present step may comprise following sub-steps.

Step 1: determining the driving device control command based on the first joint variable including the control command for first orientation joint.

Step 2: controlling the first orientation joint according to the control command for first orientation joint to align the orientation of the first operating part with the orientation of the third end effector.

The first joint variable directly represents the parameters corresponding to the first orientation joint of the operating part mapped from the third end effector before switching to controlling the imaging device. Therefore, controlling the first orientation joint based on the first joint variable aligns the orientation of the first operating part with the orientation of the third end effector, i.e., controlling the first orientation joint based on the third end effector aligns the orientation of the first operating part with the orientation of the third end effector.

In the embodiments according to the present disclosure, the first operating part can control multiple end effectors, switching control between multiple end effectors, such as switching from controlling the third end effector first to controlling the imaging device. Optionally, if the third end effector remains stationary after switching to controlling the imaging device, the first orientation joint needs to be controlled to remain stationary, thereby aligning the orientation of the first operating part with the orientation of the third end effector. If the third end effector moves after switching to controlling the imaging device, the first orientation joint needs to be controlled to move to align the orientation of the first operating part with the orientation of the third end effector.

Step S1103: controlling the first operating part and the second operating part to change the position and/or self-rotation orientation of the target point according to the operator's command.

The master console includes a first operating part and a second operating part. The first operating part can be a left-hand operating part, and the second operating part can be a right-hand operating part. The operator can change the position or orientation of the operating part according to surgical needs, thereby changing the position or orientation of the target point configured between the first operating part and the second operating part.

According to the operator's command, such as moving the left-hand operating part, or moving the right-hand operating part, or simultaneously moving both, the position of the target point is changed. Optionally, the first operating part further includes a first position joint, and the second operating part further includes a second position joint. Moving the first position joint, or moving the second position joint, or simultaneously moving both can change the position of the target point.

Optionally, the first operating part may further include a first self-rotation joint, and the second operating part may further include a second self-rotation joint. According to the operator's command, rotating the first self-rotation joint, or the second self-rotation joint, or simultaneously rotating both, can change the self-rotation orientation of the target point.

Optionally, the first operating part includes a first position joint and a first self-rotation joint, and the second operating part includes a second position joint and a second self-rotation joint. According to the operator's command, moving the first position joint, or moving the second position joint, or simultaneously moving both, can change the position of the target point; rotating the first self-rotation joint, or the second self-rotation joint, or simultaneously rotating both, can change the self-rotation orientation of the target point. By moving the position joint and self-rotation joint of the operating parts, the position and self-rotation orientation of the target point are changed.

First designated point(s) can be configured based on the first operating part, and second designated point(s) can be configured based on the second operating part. The number and position of the designated point(s) can be configured as needed. Optionally, the designated point(s) are located in the distal region of the pushable part, such as at the intersection of the rotation axes of the first self-rotation joint 125, the second self-rotation joint 126, and the third self-rotation joint 127. In other embodiments, the designated point(s) may be located in the central region of the pushable part.

Furthermore, the target point is configured based on the first designated point and the second designated point. The target point is located within a preset neighborhood range of the midpoint of the line connecting the first designated point and the second designated point. Optionally, the target point is the midpoint of the line connecting the first designated point and the second designated point.

Step S1104: the imaging device is controlled to follow the position of the target point and/or the imaging device is controlled to be aligned with the self-rotation orientation of the target point based on the mapping between the target point and the imaging device.

If position of the target point changes, the imaging device is controlled to follow the position of the target point; if self-rotation orientation of the target point changes, the imaging device is controlled to be aligned with the self-rotation orientation of the target point; if both position and self-rotation orientation of the target point change, the imaging device is controlled to follow the position of the target point and be aligned with the self-rotation orientation of the target point.

In the embodiments according to the present disclosure, a target point is provided between the first operating part and the second operating part and used as a reference to control the imaging device to follow the position of the operating part and be aligned with the self-rotation orientation of the operating part, achieving motion tracking of the imaging device controlled by the first operating part and the second operating part.

In the embodiments according to the present disclosure, after the first operating part switches to controlling the imaging device, the first orientation joint is controlled based on the first joint variable to keep the orientation of the first operating part aligned with the orientation of the third end effector. Therefore, when the first operating part switches back to controlling the third end effector, alignment is no longer required and direct manipulation can be performed, reducing the operator's waiting time and enhancing the operator's control experience.

In some embodiments according to the present disclosure, the second operating part includes a second orientation joint. Similar to the first operating part, the second operating part can control the second orientation joint based on the first joint variable to align the orientation of the second operating part with the orientation of the third end effector. The specific control process is the same as controlling the first orientation joint based on the first joint variable to align the orientation of the first operating part with the orientation of the third end effector, which will not be repeated here.

In some embodiments according to the present disclosure, after switching to controlling the imaging device, the orientations of both the left-hand operating part and the right-hand operating part are controlled to be aligned with the orientation of the third end effector, making feel of the left-hand operating part and the right-hand operating part consistent. At the same time, if it is necessary to switch back to controlling the third end effector, it provides multiple choices for the operator, who can flexibly choose the left-hand operating part or the right-hand operating part to control the third end effector, instead of having to continue using the first operating part, thus eliminating the need for alignment and enhancing the control experience.

In some embodiments according to the present disclosure, the first operating part includes a first position joint, and the second operating part includes a second position joint. To allow the operator to autonomously control the imaging device, the first operating part and the second operating part are controlled to change the position and/or self-rotation orientation of the target point based on the operator's command, specifically including:
Controlling the first operating part and the second operating part to change the position and/or self-rotation orientation of the target point based on the operator's command, and the changes in the first position joint and the second position joint do not follow the change in the third end effector.

Optionally, the position of the target point can be changed by moving the first position joint, or moving the second position joint, or simultaneously moving the first position joint and the second position joint according to the operator's command. To ensure the operator's motion autonomy and the ability to freely move the first position joint and the second position joint, it is necessary to ensure that the changes in the first position joint and the second position joint are based only on the operator's command and do not follow the change in the third end effector.

Optionally, the first position joint and the second position joint do not need to move correspondingly based on position joint variable, which means position joint variable may not be acquired, or control command for drive device is not determined based on the position joint variable even if position joint variable is acquired, or even if control command for drive device is determined based on the position joint variable, the control command for drive device is not issued to the position joint, or even if the control command for drive devices is issued to the position joint, the position joint does not respond to the command.

Optionally, the first operating part further includes a first self-rotation joint, and the second operating part includes a second self-rotation joint. According to the operator's command, the self-rotation orientation of the target point can be changed by rotating the first self-rotation joint or the second self-rotation joint, or simultaneously rotating the first self-rotation joint and the second self-rotation joint. To ensure the operator's motion autonomy and the ability to freely rotate the first self-rotation joint and the second self-rotation joint, it is necessary to ensure that the changes in the first self-rotation joint and the second self-rotation joint are based only on the operator's command and do not follow the change in the third end effector.

Optionally, the first self-rotation joint and the second self-rotation joint do not need to move correspondingly based on self-rotation joint variable, which means self-rotation joint variable may not be acquired, or even if they do, they do not determine control command for drive devices based on the self-rotation joint variable, or even if they do determine control command for drive devices based on the self-rotation joint variable, the control command for drive devices are not issued to the self-rotation joint, or even if the control command for drive devices are issued to the self-rotation joint, the self-rotation joint does not respond to the commands.

Optionally, if the operator needs to autonomously control both the position joint and the self-rotation joint at the same time, the change in the position joint does not follow the change in the third end effector, and the change in the self-rotation joint does not follow the position and orientation changes in the third end effector.

In the embodiments according to the present disclosure, the changes in the first position joint and the second position joint do not follow the change in the third end effector, and the changes in the first self-rotation joint and the second self-rotation joint do not follow the change in the third end effector. Therefore, adjusting the first orientation joint based on the third end effector is more conducive to the operator's smooth switching of controlling the third end effector and ensures the operator's motion autonomy, which is beneficial for accepting the operator's command.

In another embodiment according to the present disclosure, the second operating part includes a second orientation joint, and the operating device further includes a fourth end effector. After the first operating part controls the third end effector and the second operating part controls the fourth end effector, the first operating part and the second operating part can switch to jointly controlling the imaging device, which includes:
Acquiring the first joint variable of the first orientation joint and the third joint variable of the second orientation joint. The present step uses the same implementation principle as the aforementioned implementation principle and will not be repeated here.

Based on the mapping between the third end effector and the first operating part, the first orientation joint is controlled according to the first joint variable to align the orientation of the first operating part with the orientation of the third end effector. Based on the mapping between the fourth end effector and the second operating part, the second orientation joint is controlled according to the third joint variable to align the orientation of the second operating part with the orientation of the fourth end effector. The present step uses the same implementation principle as the aforementioned implementation principle and will not be repeated here.

The first operating part and the second operating part is controlled to change the position and/or self-rotation orientation of the target point based on the operator's command. The present step uses the same implementation principle as the aforementioned implementation principle and will not be repeated here.

Based on the mapping between the target point and the imaging device, the imaging device is controlled to follow the position of the target point and/or to be aligned with the self-rotation orientation of the target point. The present step uses the same implementation principle as the aforementioned implementation principle and will not be repeated here.

In some embodiments according to the present disclosure, after switching to controlling the imaging device, the orientation of the first operating part is aligned with the orientation of the third end effector, and the orientation of the second operating part is aligned with the orientation of the fourth end effector. Therefore, when the first operating part switches back to controlling the third end effector and the second operating part switches back to controlling the fourth end effector, neither the third end effector nor the third end effector needs to be aligned again, which reduces the operator's waiting time and enhances the operator's control experience.

In another embodiment according to the present disclosure, the first operating part further includes a first position joint and a first self-rotation joint, and the second operating part further includes a second position joint and a second self-rotation joint. The controlling the first operating part and the second operating part to change the position and/or self-rotation orientation of the target point according to the operator's command includes:
Controlling the first position joint and the second position joint to change the position and/or self-rotation orientation of the target point according to the operator's command, and the change in the first position joint does not follow the change in the third end effector. The implementation principle of the present step is the same as the aforementioned, so it will not be repeated here. The change in the second position joint does not follow the change in the fourth end effector, and the implementation principle of the present step is the same as that of the change in the first position joint not following the change in the third end effector, so it will not be repeated here.

In some embodiments according to the present disclosure, the change in the first position joint does not follow the change in the third end effector and the change in the second position joint does not follow the change in the fourth end effector, or the change in the first self-rotation joint does not follow the change in the third end effector and the change in the second self-rotation joint does not follow the change in the fourth end effector. Adjusting the first orientation joint based on the third end effector is more conducive to the operator's smooth switching control of the third end effector, and adjusting the second orientation joint based on the fourth end effector is more conducive to the operator's smooth switching control of the fourth end effector, which ensure the operator's autonomy of motion and are conducive to receiving the operator's command.

In another embodiment according to the present disclosure, the first orientation joint includes a wrist joint and a self-rotation joint. The aligning the orientation of the first operating part with the orientation of the third end effector by controlling the first orientation joint based on the mapping between the third end effector and the first operating part according to the first joint variable includes:
Step 1: the control command for drive device is determined based on the first joint variable. The control command for drive device includes control commands for wrist joint and self-rotation joint. The first joint variable includes a wrist joint variable and a self-rotation joint variable.
Step 2: the wrist joint is controlled according to the control command for wrist joint, and the self-rotation joint is controlled according to the control command for self-rotation joint, so that the orientation of the first operating part is aligned with the orientation of the third end effector.

In this case, the operator can only independently control the position joint to control the imaging device to follow the position of the target point.

In some embodiments according to the present disclosure, by controlling the wrist joint and the self-rotation joint together to align the orientation of the first operating part with the orientation of the third end effector, and controlling the imaging device according to the operator's command, so that when switching back to controlling the third end effector, there is no need to perform orientation alignment again, which simplifies the control process and improves the control experience.

Another embodiment according to the present disclosure provides a control method for a surgical robot, which includes a master console and a slave operating device. The master console includes an operating part, which includes an orientation joint, and the slave operating device includes an end effector and an imaging device. In the present embodiment, there is only one operating part for alternate one-hand control of the end effector and the imaging device. The method includes:

Obtaining joint variable of the orientation joint. The implementation principle of the present step is the same as the aforementioned step and will not be repeated here.

Controlling the orientation joint based on the mapping between the end effector and the operating part according to the joint variable, so that the orientation of the operating part is aligned with the orientation of the end effector. The orientation information of the operating part can be determined based on the pose information of specified point(s) in the operating part. The number and positions of the specified point(s) can be configured as needed. Optionally, the specified point(s) are located in the distal region of the pushable part, such as at the intersection of the rotation axes of the first self-rotation joint 125, the second self-rotation joint 126, and the third self-rotation joint 127. In other embodiments, the specified point(s) can be located in the central region of the pushable part. The number of specified point(s) can be one or more. The orientation of the operating part can include orientation angle information of the operating part based on at least one coordinate axis of the coordinate of the display device.

Controlling the operating part to change position and/or self-rotation orientation according to the operator's command. The implementation principle of the present step is the same as the aforementioned step and will not be repeated here.

Controlling the imaging device to follow the position of the operating part and/or controlling the self-rotation orientation of the imaging device to be aligned with the operating part based on the mapping between the operating part and the imaging device. The implementation principle of the present step is the same as the aforementioned step and will not be repeated here.

Further, the operating part further includes a position joint. The controlling the operating part to change position according to the operator's command includes: controlling the position joint to change position according to the operator's command, and the change in the position joint does not follow the change in the end effector. The implementation principle of the present step is the same as the aforementioned step and will not be repeated here.

Further, the operating part further includes a self-rotation joint. The controlling the operating part to change self-rotation orientation according to the operator's command includes: controlling the self-rotation joint to change self-rotation orientation according to the operator's command, and the change in the self-rotation joint does not follow the change in the end effector. The implementation principle of the present step is the same as the aforementioned step and will not be repeated here.

Further, the operating part further includes a position joint and a self-rotation joint. The controlling the operating part to change position and self-rotation orientation according to the operator's command includes: controlling the position joint to change position according to the operator's command, and the change in the position joint does not follow the change in the end effector; controlling the self-rotation joint to change self-rotation orientation according to the operator's command, and the change in the self-rotation joint does not follow the change in the end effector. The implementation principle of the present step is the same as the aforementioned step and will not be repeated here.

In some embodiments according to the present disclosure, only one operating part is provided for alternate one-hand control of the end effector and the imaging device. When the operating part switches back to controlling the end effector, there is no need to perform alignment again by aligning the orientation of the operating part with the orientation of the end effector, which reduces the operator's waiting time and improves the operator's control experience.

In another embodiment according to the present disclosure, the operating part of the surgical robot can include not only the first operating part and the second operating part but also a larger number of operating parts. The number of operating parts can be flexibly configured according to the actual surgical situation, and the number of end effectors of the slave operating device can be flexibly configured according to the actual surgical situation. Each end effector can have different degrees of freedom of motion. In this control situation, the operating part can freely choose which end effector to follow, which orientation joints to adjust, and whether to use one hand or both hands to control the imaging device, thereby meeting the needs of personalized complex surgeries.

In some embodiments according to the present disclosure, a surgical robot is provided, which includes a master console comprising an operating part, a slave operating device, and a controller coupled with the operating part and the slave operating device and configured to execute the control method for the surgical robot described above.

In some embodiments according to the present disclosure, a computer-readable storage medium is provided. The computer-readable storage medium stores computer program which implements each process of the embodiments of the control method for the surgical robot described above and achieves the same technical effect when executed by a processor. To avoid repetition, details are not described here. The computer-readable storage medium includes, for instance, a Read-Only Memory (ROM), a Random Access Memory (RAM), a magnetic disk, or an optical disk.

The technical features of the embodiments described above can be combined arbitrarily. For simplicity of description, not all possible combinations of the technical features in the embodiments described above are described. However, as long as there is no contradiction in the combination of these technical features, they should all be considered within the scope described in this specification.

The embodiments described above only express several implementation modes of the present disclosure, which are described more specifically and detailedly. However, they should not be construed as limiting the scope of the invention patent. It should be pointed out that for ordinary technicians in this field, several deformations and improvements can still be made without departing from the concept of the present disclosure, which all belong to the protection scope of the present disclosure. Therefore, the protection scope of the patent of the present disclosure shall be subject to the appended claims.

## Claims

1. A surgical robot, comprising:
an input device;
a robotic arm, on which an instrument is detachably mounted; and
a controller configured to:
suspend control of the instrument by the input device in response to a disconnecting command for master-slave control, and control motion of the input device when the robotic arm is actuated to change an orientation of an end effector of the instrument so that an orientation of the input device follows the orientation of the end effector.

2. The surgical robot of claim 1, wherein the robotic arm comprises a parallelogram mechanism and an instrument-holding arm, the instrument is detachably mounted on the instrument-holding arm, and a long shaft of the instrument passes through a remote center of motion defined by the parallelogram mechanism; and
the controller is configured to control motion of the input device when the parallelogram mechanism is actuated to rotate the long shaft of the instrument about the remote center of motion so that the orientation of the input device follows the orientation of the end effector of the instrument.

3. The surgical robot of claim 2, wherein the robotic arm further comprises an adjustment arm, a distal end of the adjustment arm is connected to a proximal end of the parallelogram mechanism, and the controller is configured to control motion of the input device when the adjustment arm is actuated to change an orientation of the remote center of motion so that the orientation of the input device follows the orientation of the end effector.

4. The surgical robot of claim 1, wherein the controller is further configured to resume control of the instrument by the input device in response to an activation command for the master-slave control, and the controller stops controlling the orientation of the input device to be aligned with the orientation of the end effector of the instrument after activation of the master-slave control.

5. A surgical robot, comprising:
an input device and a display device;
a first robotic arm and a second robotic arm, an instrument detachably mounted on the first robotic arm, an imaging device detachably mounted on the second robotic arm; and
a controller configured to:
suspend control of the instrument by the input device in response to a disconnecting command for master-slave control; and
control motion of the input device when the second robotic arm is actuated to change an orientation of an end effector of the instrument relative to the imaging device so that an orientation of the input device relative to the display device follows the orientation of the end effector relative to the imaging device.

6. The surgical robot of claim 5, wherein the second robotic arm comprises a parallelogram mechanism and an instrument-holding arm, the imaging device is detachably mounted on the instrument-holding arm, and a long shaft of the imaging device passes through a remote center of motion defined by the parallelogram mechanism; and
the controller is configured to control motion of the input device when the parallelogram mechanism is actuated to rotate the long shaft of the imaging device about the remote center of motion so that the orientation of the input device relative to the display device follows the orientation of the end effector relative to the imaging device.

7. A surgical robot, comprising:
an input device;
a first robotic arm and a second robotic arm, a first instrument detachably mounted on the first robotic arm, a first instrument detachably mounted on the second robotic arm;
a controller configured to:
switch the input device from controlling the first instrument to controlling the second instrument in response to an instrument-switching command, and control motion of the input device to align an orientation of the input device with an orientation of an end effector of the second instrument.

8. A surgical robot, comprising:
an input device;
an instrument; and
a controller configured to:
suspend control of the instrument by the input device in response to a disconnecting command for master-slave control;
determine whether alignment between the input device and an end effector of the instrument has been performed after initialization of an alignment state between the input device and the instrument; and
control an orientation of the input device to follow an orientation of an end effector of the instrument when the alignment has been performed;
control the orientation of the input device to be aligned with the orientation of the end effector of the instrument when the alignment has not been performed.

9. The surgical robot of claim 8, wherein the controller is further configured to resume control of the instrument by the input device in response to an activation command for the master-slave control, and the controller stops controlling the orientation of the input device to be aligned with the orientation of the end effector of the instrument after activation of the master-slave control.

10. The surgical robot of claim 8, wherein the input device comprises multiple motors, and the controller is configured to control the multiple motors to rotate in an accelerated manner to move the input device so that the orientation of the input device follows the orientation of the end effector; or the controller is configured to control the multiple motors to rotate at a uniform speed to move the input device so that the orientation of the input device is aligned with the orientation of the end effector of the instrument.

11. A surgical robot, comprising:
an input device, comprising a wrist joint assembly configured to change an orientation of the input device and an elbow joint assembly configured to change a position of the input device;
an instrument; and
a controller configured to:
suspend control of the instrument by the input device in response to a disconnecting command for master-slave control; and
control motion of the wrist joint assembly in response to motion of the elbow joint assembly or a change in an orientation of the input device so that the orientation of the input device follows an orientation of an end effector of the instrument.

12. The surgical robot of claim 11, wherein the controller is configured to control a motor driving the wrist joint assembly to rotate in an accelerated manner so that the orientation of the input device follows the orientation of the end effector of the instrument.

13. A control method for a surgical robot, wherein the surgical robot comprises a master console and a slave operating device, the master console comprises a first operating part, the first operating part comprises multiple sets of master joints, the slave operating device comprises a first instrument with a first end effector, and the control method comprises:
determining first slave pose information of the first end effector based on first master pose information of the first operating part according to a mapping between the first operating part and the slave operating device;
controlling the first end effector to adjust pose of the first end effector based on the first slave pose information, and acquiring second slave pose information of the first end effector;
determining second master pose information of the first operating part based on the second slave pose information according to the mapping between the first operating part and the slave operating device;
controlling a first set of master joints among the multiple sets of master joints based on the second master pose information to adjust a pitch and yaw orientations of the first operating part to be aligned with the first end effector; and
controlling a second set of master joints among the multiple sets of master joints based on an operator's command to adjust at least one of a position of the first end effector to follow a position of the first operating part and a self-rotation orientation of the first end effector to be aligned with a self-rotation orientation of the first operating part.

14. The control method of claim 13, wherein the slave operating device comprises a wrist joint drive device configured to controlling wrist joint motion of an instrument with a wrist joint, the controlling the first end effector to adjust the pose of the first end effector based on the first slave pose information comprises:
transmitting an operation command to the wrist joint drive device, the operation command indicating the wrist joint drive device to remain stationary; or
not transmitting an operation command to the wrist joint drive device, so that the wrist joint drive device remains stationary.

15. The control method of claim 13, wherein the controlling the second set of master joints among the multiple sets of master joints based on the operator's command comprises:
controlling the second set of master joints among the multiple sets of master joints based on the operator's command, wherein change in the second set of master joints does not follow change in the first end effector.

16. A control method for a surgical robot, wherein the surgical robot comprises a master console and a slave operating device, the master console comprises a first operating part and a second operating part, a target point is provided between the first operating part and the second operating part, the first operating part comprises a first orientation joint, the slave operating device comprises a third end effector and an imaging device, and the control method comprises:
acquiring a first joint variable of the first orientation joint;
controlling the first orientation joint based on the first joint variable to align an orientation of the first operating part with an orientation of the third end effector according to a mapping between the third end effector and the first operating part;
controlling the first operating part and the second operating part to change at least one of a position and a self-rotation orientation of the target point based on an operator's command; and
controlling the imaging device to at least one of follow the position of the target point and be aligned with the self-rotation orientation of the target point based on a mapping between the target point and the imaging device.

17. The control method of claim 16, wherein the second operating part comprises a second orientation joint, the slave operating device further comprises a fourth end effector, and the method further comprises:
acquiring a third joint variable of the second orientation joint; and
controlling the second orientation joint based on the third joint variable to align an orientation of the second operating part with an orientation of the fourth end effector according to a mapping between the fourth end effector and the second operating part.

18. The control method of claim 16, wherein the first operating part further comprises a first position joint, the second operating part further comprises a second position joint, and the controlling the first operating part and the second operating part to change at least one of the position and the self-rotation orientation of the target point based on the operator's command comprises:
controlling the first position joint and the second position joint to change at least one of the position and the self-rotation orientation of the target point based on the operator's command, wherein changes in the first position joint and the second position joint do not follow change in the third end effector.

19. The control method of claim 17, wherein the first operating part further comprises a first position joint, the second operating part further comprises a second position joint, and the controlling the first operating part and the second operating part to change at least one of the position and the self-rotation orientation of the target point based on the operator's command comprises:
controlling the first position joint and the second position joint to change at least one of the position and the self-rotation orientation of the target point based on the operator's command, wherein change in the first position joint does not follow change in the third end effector, and change in the second position joint does not follow change in the fourth end effector.

20. A control method for a surgical robot, wherein the surgical robot comprises a master console and a slave operating device, the master console comprises an operating part, the operating part comprises an orientation joint, the slave operating device comprises an end effector and an imaging device, and the method comprises:
acquiring a joint variable of the orientation joint;
controlling the orientation joint based on the joint variable to align an orientation of the operating part with an orientation of the end effector according to a mapping between the end effector and the operating part;
controlling the operating part to change at least one of a position and a self-rotation orientation of the operating part based on an operator's command; and
controlling the imaging device to at least one of follow the position of the operating part and be aligned with a self-rotation orientation of the operating part based on a mapping between the operating part and the imaging device.
